# EUROPEAN PATENT APPLICATION

(11) **EP 3 862 034 A1**
(43) Date of publication of application: **11.08.2021**
(21) Application number: 20155972.1
(22) Date of filing: 06.02.2020
(51) Int. Cl.: A61M 1/10, A61M 1/12

(54) **BLOOD PUMP PLACEMENT AND INTRAVASCULAR BLOOD PUMP**

(71) Applicant: Abiomed Europe GmbH, 52074 Aachen (DE)
(72) Inventor: SIESS, Thorsten, 52074 Aachen (DE); KERKHOFFS, Wolfgang, 52074 Aachen (DE); DA SILVA RODRIGUES, Cristine, 52074 Aachen (DE); KEYSSELITZ, Ellen, 52074 Aachen (DE)
(74) Representative: Klunker IP Patentanwälte PartG mbB

(57) **Abstract**

An intravascular blood pump for percutaneous insertion into a patient's vasculature comprises a pumping device (30) and a supply catheter (36). The pumping device comprises a pump section with a blood flow inlet, blood flow outlet, and impeller for conveying blood from the inlet to the outlet and further comprises a drive section connected to the pump section and adapted to drive the impeller. The supply line supplies the drive section with electric energy for driving the impeller. An anchoring structure (60; 70) is provided at a distal end region of the intravascular blood pump. A connecting catheter may be attached to the anchoring structure from distally in order to guide the intravascular blood pump through the patient's vasculature in a distal direction.

## Description

### FIELD OF THE INVENTION

This invention relates to methods of placing an intravascular blood pump in a patient for assisting the left ventricle of the patient's heart, placement tools for use in such methods and an intravascular blood pump which is particularly suitable for the placement methods.

Thus, the invention generally relates to intravascular blood pumps. In contrast to extracorporeal blood pumps or blood pumps that are placed in the abdomen, thoracic cavity or other body cavity of a patient, intravascular blood pumps are placed in the blood vessel, such as in the patient's heart, the latter also being referred to as "intracardiac" blood pumps. The invention is particularly, but not exclusively, related to intravascular blood pumps that are percutaneously inserted into the patient's vascular system.

### BACKGROUND OF THE INVENTION

Intravascular blood pumps are known to support the function of a patient's heart, either in a short-term application, in which the blood pump is implanted in the patient for a couple of days or weeks, or a long-term application, in which the blood pump is implanted in the patient for a couple of weeks or months (e.g. six months or longer) up to e.g. two years. Different types of intravascular blood pumps are known, such as axial blood pumps, centrifugal blood pumps or diagonal blood pumps, which are also sometimes referred to as mixed-type blood pumps. These types of intravascular blood pumps may be inserted into a patient's vascular system by means of a catheter through a percutaneous incision.

An intravascular blood pump typically comprises a pump section having a blood flow inlet and a blood flow outlet and an impeller rotatable about an axis of rotation and sized and shaped for conveying blood from the blood flow inlet to the blood flow outlet. An intravascular blood pump further comprises a drive section for driving the impeller. Hereinafter, the pump section and the drive section together are referred to as a "pumping device". Further, an intravascular blood pump typically comprises a supply catheter connected to the pumping device with a proximal end of the catheter extending out of the patient's body. Herein, "proximal" refers to a position closer to the surgeon or a direction towards the surgeon, whereas "distal" refers to a position remote from the surgeon or a direction away from the surgeon into the patient. The supply catheter may enclose any supply lines for the pumping device, such as electric lines, purge fluid lines, sensor lines, a drive cable etc.

In cases where the impeller is driven by an internal motor, the pumping device may include both the pump section and the drive section in a common housing, the pump section comprising the blood flow inlet, the blood flow outlet and the impeller, and the drive section being axially attached to the pump section and comprising the motor for driving the impeller. Electric energy for driving the motor is supplied via supply lines running through the supply catheter. In cases where the impeller is driven by an external motor situated outside the patient's body, the supply catheter may enclose a bendable drive cable as part of the drive section, the drive cable connecting the external motor to the impeller and supplying kinetic energy to the impeller.

While the placement methods described herein may also be suitable for cable driven blood pumps as described above, preferred in this context are intravascular blood pumps which include both the pump section and the drive section attached together so as to form an integral, fully implantable pumping device.

Typically, an intravascular blood pump acting as a left ventricular assist device (LVAD) is inserted through a femoral artery and through the aorta into the left ventricle of the patient's heart. However, sometimes the inner diameters of arterial vessels are reduced due to calcification, whereas such problem does usually not arise in venous vessels. In this situation, placement of the blood pump through the femoral artery may be impossible. In other situations, the blood pump may be too large to be introduced even through non-calcified arterial vessels. For instance, long-term devices may be relatively bulky and large in diameter (e.g. 16 French or more, up to 18 French) and thus difficult to insert via the arterial side. Therefore, other insertion techniques have been described, in which the blood pump is inserted via the venous side having relatively large inner diameters, e.g. through a femoral vein, and further through a perforation of the atrial septum into the left ventricle. Introducing the blood pump through the femoral vein has the further advantage that due to the very low blood pressure on the venous side of the ventricular system, the risk of blood leaking out of the femoral vein through the entrance point is substantially reduced.

It is known for instance from US 2006/0155158 A1 to use two guide wires that are inserted from the venous side and arterial side, respectively, and magnetically connected inside the patient's heart to form a continuous guide wire for pulling the blood pump into the femoral vein and further through the vena cava and the perforation in the atrial septum into the left ventricle. However, finding and securely connecting the two guide wires in the patient's heart is problematic.

Another problem arises in long-term applications, where it is desirable that the patient is mobile, for instance allowing the patient to leave the bed and walk around. That is, when the blood pump is inserted in a transfemoral approach, i.e. inserted via a femoral artery or femoral vein accessed via a small incision in the groin, mobility of the patient is limited because the supply catheter percutaneously exits the patient in the groin, which makes walking difficult.

### SUMMARY OF THE INVENTION

The present invention therefore relates to the placement of an intravascular blood pump in the patient's heart so as to function as a LVAD. In this context, further disclosed herein are an intravascular blood pump as well as placement tools which are particularly suitable for use in the disclosed placement methods.

A method of placing an intravascular blood pump of the type as described above in a patient comprises, according to a first principle, the steps of placing a first guide wire through the patient's vascular system, including through the patient's heart, such that it extends out of the patient's body with a first end through a first percutaneous access on an arterial side of the vascular system, such as through the axillary artery or subclavian artery, and with a second end through a second percutaneous access on a venous side of the vascular system, and then using the first guide wire in the further procedure of placing the intravascular blood pump into the patient's body through the second percutaneous access, i.e. from the venous side of the patient's vascular system.

Placing the first guide wire all the way through the patient's vascular system such that it extends out of the patient's body from both a vein and an artery may be achieved in the following way. First, a transseptal sheath is inserted from the venous side of the patient's vascular system through a perforation created in a septum of the patient's heart into the left side of the heart, wherein the septal perforation is preferably a perforation of the atrial septum. Then, the first end of the first guide wire is advanced through the transseptal sheath into the left part of the heart. Thereafter, the transseptal sheath may be pulled back. Second, the passage through the septum may be expanded, for instance by means of a PTA balloon catheter. Third, an introducer sheath is advanced through the septum such that a front part of the introducer sheath extends into the left part of the heart, preferably into the left ventricle. This process may advantageously be supported by the PTA balloon catheter. In the case that a PTA balloon catheter has been used, this may now be withdrawn from the vascular system. Fourth, a balloon catheter, such as a Swan-Ganz catheter, is advanced through the introducer sheath into the left part of the heart, preferably into the left ventricle. Then, the balloon of the balloon catheter is inflated and the first end of the first guide wire is guided towards and out of the first percutaneous access with the aid of the balloon catheter following the patient's blood flow. As a result, the first guide wire now passes through the patient's vascular system, including the heart, and extends out of the patient's body with both of its ends.

Preferably, prior to inflating the balloon of the Swan-Ganz catheter, a second guide wire may be advanced through an arterial access (the above mentioned "first" percutaneous access) into the left part of the heart, preferably into the left ventricle, and the balloon catheter may be guided along a direction which is indicated by the second guide wire towards and out of the arterial access.

Further preferably, when the transseptal sheath is inserted at the beginning of the procedure, the transseptal sheath may be inserted through a percutaneous access into a vein below the vena cava inferior, preferably into the femoral vein which may be accessed by the known Seldinger technique typically in the patient's groin. Advancing a transseptal sheath and guide wire through the atrial septum into the left part of the heart, in particular up into the left ventricle, is relatively easy coming from the vena cava inferior because of the natural curvature within the human ventricular system, including the curvature in the heart. The percutaneous access into a vein below the vena cava inferior may be different to the above mentioned "second" percutaneous access and, thus, may be referred to as a "third" percutaneous access. In this case, a snare catheter may be inserted through the second percutaneous access into a vein above the vena cava superior, such as into the subclavian vein or axillary vein, and advanced through the vena cava superior towards the first guide wire in the vena cava inferior, where it is used to catch the first guide wire and move the second end of the first guide wire into the third percutaneous access and out of the second percutaneous access.

Once the first guide wire has been placed to extend with both ends out of the ventricular system, there are various options of placing the intravascular blood pump in the left part of the heart with the aid of the first guide wire. A first option may comprise the following steps. First, a front end of a coupling catheter is attached to the first end of the first guide wire, preferably in a form-fitting manner, and guided through the patient's vascular system, including through the patient's heart, using the first guide wire until the front end of the coupling catheter extends through the second percutaneous access out of the patient's body. Preferably, the balloon catheter is withdrawn from the patient's body while the coupling catheter is advanced. Second, a distal end of the intravascular blood pump is coupled to the coupling catheter, preferably in a form-fitting manner, and advanced through the patient's vascular system, including through the atrial septum, so as to place its pump section into the left part of the heart, preferably across the aortic valve. In this configuration, the supply catheter of the intravascular blood pump, which extends through the heart into the left ventricle, forms a loop in the left ventricle and may come to lie against the heart wall, thereby supporting the blood pump and preventing that the blood pump moves into the left ventricle during its operation.

Optionally, the intravascular blood pump may be anchored in a wall of a blood vessel, such as in the aortic wall. This may be achieved by moving a plurality of spikes from a circumference of the intravascular blood pump from a radially collapsed configuration to a radially expanded configuration to engage the wall of the blood vessel. In particular, the expandable spikes may partially penetrate the vessel wall. The spikes may have a stop to prevent them from penetrating too deeply into the vessel wall. Third, the coupling catheter is decoupled from the distal end of the intravascular blood pump and withdrawn from the patient's body. Finally, the introducer sheath may be withdrawn from the patient's body.

A second option of placing the intravascular blood pump in the left part of the heart with the aid of the first guide wire, according to the first placement principle, may comprise the following steps. First, the balloon catheter is withdrawn from the patient's body while keeping the first end of the first guide wire extending through the second percutaneous access out of the patient's body. Second, the introducer sheath is guided along the first guide wire through the patient's vascular system, including through the patient's heart, until the front end of the introducer sheath extends through the second percutaneous access out of the patient's body. Third, the second end of the first guide wire is fed through a loop provided at a distal end of the intravascular blood pump and is advanced through the introducer sheath until the second end of the first guide wire extends out of the front end of the introducer sheath. Fourth, the first end second ends of the first guide wire extending through the first percutaneous access out of the introducer sheath are gripped and used to advance the intravascular blood pump along the introducer sheath through the patient's vascular system, including through the septum, so as to place its pump section into the left part of the heart, preferably across the aortic valve. Fifth, the first guide wire is released or unfed from the loop provided at the distal end of the intravascular blood pump and withdrawn from the introducer sheath. Finally, the introducer sheath may be withdrawn from the patient's body. Again, the intravascular blood pump may be anchored in a wall of a blood vessel, such as in an aortic wall, for instance by moving a plurality of spikes from a circumference of the intravascular blood pump from a radially collapsed configuration to a radially expanded configuration to engage the wall of the blood vessel. As mentioned above, the expandable spikes may partially penetrate the vessel wall and may have a stop to prevent them from penetrating too deeply into the vessel wall.

In all aspects of the first placement principle described above, the supply line may comprise a drive cable connecting the pump section with an external motor and providing the pump section with kinetic energy. The pump section may even comprise an expandable rotor. However, the described placement principles are particularly suitable for non-expandable blood pumps which require relatively large vascular cross-sections to be advanced towards and into the heart, as provided e.g. by the venous vasculature. It is particularly preferable to employ intravascular blood pumps with a direct drive, i.e. blood pumps in which a drive section is axially connected to and implanted along with the pump section, because a long, flexible drive cable is not particularly suitable for being placed along the windy path all the way into the left ventricle. In this case, electric energy may be supplied to the drive section via the supply lines.

Arranging the supply catheter or supply line not on the arterial side but on the venous side of the patient's vasculature may be advantageous for long-term applications, in which tissue of an arterial vessel would tend to grow into the supply catheter. Also, with this arrangement, the supply catheter or supply line does not hinder the arterial blood flow at all, so that an accordingly higher blood flow volume can be achieved with an intravascular blood pump placed in this way.

According to a second principle of a method of placing an intravascular blood pump in a patient, a blood pump may be inserted via a transapical access. The method may comprise the step of accessing the patient's thoracic cavity by puncturing the wall of the patient's heart in the apex of the heart to access the left ventricle of the heart. The access to the patient's thoracic cavity is preferably performed in a minimally invasive procedure. The method further comprises the steps of advancing the pumping device, i.e. the pump section including the drive section, through the puncture in the apical wall into the left ventricle with the distal end of the pumping device ahead, advancing the pumping device through the left ventricle and the aortic valve towards the aorta, and placing the blood pump such that the blood flow inlet is disposed in the left ventricle, the blood flow outlet is disposed in the aorta, and the drive section is also disposed in the aorta, whereas the supply catheter extends through the puncture in the apex of the heart and percutaneously exits the patient's body. This requires that the pump section of the intravascular blood pump is arranged axially between the drive section and the supply catheter or supply line, as will be further explained hereinafter.

Also the placement method according to the second principle may further comprise the step of securing a position of the blood pump within the patient's body by means of an anchoring device that engages an inner wall of the patient's aorta. This is particularly useful because the intravascular blood pump tends to move from the aorta into the left ventricle. This may be caused by movements of the patient as well as by the pumping pressure of the blood pump pushing the blood pump backwards into the left ventricle. Although, this is less of a problem if the supply catheter extends through the aortic arch, thereby providing some fixation, it may still be advantageous.

As mentioned above, the distal end region of the intravascular blood pump is preferably provided with an anchoring structure, wherein the intravascular blood pump further comprises as minimum components a pumping device and a supply line, wherein the pumping device comprises a pump section with an impeller and a drive section for driving the impeller, whereas the supply line supplies the energy for driving the impeller.

The anchoring structure may comprise, as already mentioned, at least one anchor adapted to anchor the intravascular blood pump in a wall of the patient's blood vessel, such as in the patient's aorta, wherein the anchor may comprise spikes that are expandable and collapsible.

However, the intravascular blood pump may further or alternatively comprise an entirely different anchoring structure, namely for use in the above mentioned placement methods in order to connect the distal end of the intravascular blood pump to the coupling catheter according to the first option of the first placement principle or to the first guide wire according to the second option of the first placement principle.

According to a first preferred embodiment, such anchoring structure comprises a hook or a loop at a distal end of the intravascular blood pump. The coupling catheter may then comprise a corresponding hook to hook into the hook or loop of the blood pump or a loop into which the hook of the blood pump is hooked. In one form, the loop may be made from a soft, elastic material so as to form an atraumatic distal extension at the axial end of the intravascular blood pump. The typical pigtail-shaped or J-shaped atraumatic distal extensions may be replaced by such loop. Alternatively, the conventional atraumatic distal extensions may be provided with a loop, preferably at a distal end thereof. While the blood pump is guided through the patient's vasculature by both pushing the blood pump forward and slightly pulling the other end using the coupling catheter, the J-type or pigtail-shaped atraumatic extension may stretch and, after release, recoil.

In a second preferred embodiment, such anchoring structure may comprise a neck-and-head structure at an axial end of the end region of the intravascular blood pump. In this case, the coupling catheter may comprise a gripper to connect to the neck-and-head structure. Preferably, the connector has a gripper with a first grip and a second grip adapted to grip around the head of the neck-and-head structure. The same gripper may likewise be used to close around the hook or loop of the intravascular blood pump if it is provided with a hook or loop instead of the neck-and-head structure. The neck-and-head structure may distally extend from the axial end of the end region of the intravascular blood pump. Alternatively, the neck-and-head structure may be provided somewhat hidden within a frontal cavity of the intravascular blood pump in order to avoid that it may damage tissue.

Preferably, a connecting surface between a head and a neck of the neck-and-head structure is inclined by 90° relative to a longitudinal axis of the intravascular blood pump, or the connecting surface may even be inclined by more than 90° relative to the longitudinal axis of the intravascular blood pump so as to form an undercut. In order to provide a perfect fit between the perpendicular or even negatively inclined connecting surface, the gripper surface is likewise inclined by 90° relative to the longitudinal axis of the connecting catheter, or even by more than 90° so as to form and undercut mating with the undercut of the neck-and-head structure. This way, the forces acting on the head, more specifically acting on the connecting surface between the head and neck when the blood pump is cautiously pulled through the vasculature using the connecting catheter, are purely axial forces so that any forces to keep the first and second grips closed behind the head may be minimal. Those closing forces are acting radially in a direction perpendicular towards the longitudinal axis of the blood pump and, therefore, any pulling forces acting on the connecting surface when the blood pump is guided through the patient's vasculature will have no force component opposing the closing forces of the gripper. The gripper surfaces in conventional connecting catheters are typically rounded and, therefore, disadvantageous.

Different types of drives may be used. Preferably, the drive may comprise a stator including a plurality of posts, each post having a coil winding that can be controlled to create a rotating magnetic field. The impeller then may have a corresponding plurality of permanent magnets to couple to the rotating magnetic field for causing rotation of the impeller. Alternatively, the drive may comprise a rotor driven by an electric motor, the rotor including a plurality of permanent magnets, wherein the impeller comprises a corresponding plurality of permanent magnets that are magnetically coupled to the magnets of the rotor to form a magnetic coupling that is configured to transfer a rotation of the rotor to the impeller. In both drives the electric components are enclosed by a housing and do not have blood contact. The rotating movement of the electric motor is magnetically transferred to the impeller. However, other types of drives may likewise be used, such as drives purged with a purge fluid, such as a saline solution.

It will be appreciated that the intravascular blood pump is preferably a left ventricular assist device (LVAD), which is configured to pump blood from the left ventricle into the aorta. The blood flow inlet may be arranged at the proximal end of the pumping device and the blood flow outlet may be arranged at the distal end of the pumping device. This may be referred to as a "forcing pump" as the blood is forced in a direction away from the surgeon, i.e. away from the supply catheter. This applies for applications in which the supply catheter is placed on the venous side of the patient's vascularity. Vice versa, the blood flow inlet may be arranged at the distal end of the pumping device and the blood flow outlet may be arranged at the proximal end of the pumping device. This may be referred to as a "sucking pump" as the blood is sucked in a direction towards the surgeon, i.e. towards the supply catheter. This applies for applications in which the supply catheter is placed on the arterial side of the patient's vasculature.

Preferably, the intravascular blood pump is a forcing pump, more preferably a forcing pump in which the pump section is arranged axially between the drive section and the supply catheter or supply line. In prior art intravascular blood pumps having the pump section and the drive section integrated as a fully implantable pumping device, the arrangement is different, i.e. the pump section is arranged axially between the drive section and the supply catheter. While these prior art intravascular blood pumps are typically operated as sucking pumps, the rotation of the impeller may be reversed so that the same blood pump may likewise be used as a forcing pump. However, since the main application of such blood pumps is for use as sucking pumps, the flow characteristics are not as good when the same blood pump is used as a forcing pump. This is particularly the case where the pump section is not designed as a pure axial pump but includes at least a radial component, i.e. it is formed as a diagonal or mixed-type pump or as a centrifugal pump. The radial component of the pump has the effect of a centrifugal pump and, therefore, contributes to the kinetic energy which is transferred from the pump into the blood flow. When such pumps are operated in the reverse direction, such radial component is not only lost for the generation of kinetic energy, but it is even counterproductive. This loss in performance must either be accepted or compensated by a higher rotational speed of the impeller, but there are certain limits to the rotational speed.

A preferred design of a forcing blood pump according to the present invention therefore comprises a pumping device and a supply line. The pumping device comprises a pump section in the usual way, i.e. the pump section has a blood flow inlet, a blood flow outlet and an impeller rotatable about an axis of rotation for conveying blood from the blood flow inlet to the blood flow outlet. The pumping device further comprises a drive section connected to the pump section and adapted to drive the impeller. Preferably, the drive section is axially connected to and implanted along with the pump section, as is generally known in the art. The supply line is adapted to supply the drive section at least with electric energy for driving the impeller. Accordingly, the supply line includes or consists at least of an electric cable. However, the supply line is preferably formed as a supply catheter which may include the electric cable as well as further components, such as a pressure line, e.g. in the form of one or more glass fibers. Importantly, the pump section is arranged along the axis of rotation between the drive section and the supply line or supply catheter. Thus, the supply line or supply catheter is attached to the pump section rather than to the drive section.

If this intravascular blood pump is placed in the patient's vasculature such that the blood flow inlet is located in the patient's heart and the blood flow outlet is located in the aorta, the blood pump can be operated as a forcing pump, i.e. in the direction away from the supply line or supply catheter and, thus, away from the surgeon, providing the advantage that the supply line or supply catheter does not run through the arterial vasculature and, therefore, does not hinder the blood flow which is supported or created by the blood pump. Accordingly, a higher blood flow volume can be achieved in this manner, independent of the type of the blood pump. A particular advantage arises when the blood pump has a radial component, such as in axial-radial blood pumps, diagonal blood pumps or centrifugal blood pumps. Here, since the drive section is positioned distally of the pump section, the impeller driven by the drive section may be arranged close to the blood flow outlet and deliver the blood through the blood flow outlet radially or at least with a radial component, so that the blood pump is able to provide a relatively high blood flow volume.

The energy for driving the impeller is preferably transferred in a conductive manner from the supply line across the pump section to the drive section. Accordingly, an electrically conductive connection may be provided to extend along the pump section in order to electrically connect the supply line with a motor of the drive section.

Intravascular blood pumps of the type described above typically comprise a cannula as part of the pump section, and such cannula may be provided also in the intravascular blood pump described here. The cannula is usually the part of the pump section which extends through the aortic valve, in some applications through both the aortic valve and the mitral valve. In order for the blood pump to be maneuverable through the patient's vascular system, the cannula is bendable along its longitudinal axis. The cannula may be precurved in the shape which it shall assume when it is properly installed in the patient's heart.

While the blood flow inlet of the pump section is preferably arranged at the distal end of the cannula close to the supply line or supply catheter, the blood flow outlet of the pump may be arranged at a distal end of the cannula, i.e. next to the motor section. Thus, the impeller driven by the motor in the drive section is then likewise located next to the drive section so as to be able to force the blood out of the pump section directly through the blood flow outlet.

Since the cannula undergoes substantial bending when it is maneuvered through the veins and the heart into place across the aortic valve, specific measures have to be taken to prevent that the electrically conductive connection between the supply line and the drive section across the pump section does not rupture during such maneuvers. For instance, corresponding electric lines may be run loosely through the lumen of the cannula. But this is not preferred because such arrangement may cause turbulences and blood clotting. Rather, it is preferred to arrange the electric lines along a wall of the cannula.

For instance, either the stranded wires of the electric cable of the supply line or specific stranded wires or single wires, such as e.g. the motor cables of the motor in the drive section which usually carry an insulation of a polymeric lacquer, may extend along the wall of the cannula, preferably in a neutral bending plane of the cannula. The neutral bending plane of the cannula is the plane along which the cannula is most likely to bend, e.g. because other regions of the cannula are made to elongate under less force as compared to the area of the cannula in the region of the neutral bending plane. For instance, the neutral bending plane of the cannula may be predetermined by a central plane of curvature of the cannula, which corresponds to the curvature that the cannula is supposed to assume when properly placed in the patient's heart.

For instance, the electric lines may extend along the wall of the cannula in a strictly longitudinal direction parallel to the longitudinal axis of the cannula, preferably along the mutual bending plane, and may be stretchable in the longitudinal direction so as to prevent that they rupture by elongation.

Alternatively, the electric lines may extend along the wall of the cannula inside at least one bendable tube. The bendable tube is preferably disposed along the mutual bending plane, as explained before, but this is not a requirement when the electric lines are laid with slack inside the tube.

One bendable tube may be provided for each one of the electric lines, or a plurality of the electric lines may run through the bendable tube or tubes.

In a specific embodiment, a plurality of the bendable tubes is provided on opposite sides of the wall of the cannula along the longitudinal axis of the cannula so as to create a mutual bending plane within the cannula. For instance, there may be one bendable tube provided on opposite sides of the cannula with two electric lines running through one tube and a third electric line together with an optical fiber of a pressure sensor running through the other tube. Alternatively, two tubes may be arranged on each side of the wall, one tube for each line. In the context of the electric lines (or other lines) arranged on opposite sides of the wall of the cannula, it is advantageous to provide at least the blood flow inlet with an even number of inlet ports so that the electric lines arranged on opposite sides of the cannula may continue straight across the bridges separating neighboring inlet ports.

Preferably, the bendable tube is made of a shape memory alloy, such as nitinol.

Further alternatively, the electric lines may extend meander-like along the wall of the cannula. This has the effect that an elongation of the wall of the cannula resulting from bending the cannula is much higher than the elongation in the meander-like extending electric lines. This effect is achieved even where the meander-like extending electric lines are not arranged along a neutral bending plane of the cannula, which may, as mentioned above, be predetermined by a central plane of curvature of the cannula. In particular, when the electric lines are not entirely fixed to the wall of the cannula but are allowed to shift marginally, bending of the cannula may not cause any elongation of the meander-like extending electric lines at all. For instance, the meander-like extending electric lines may be fixed in or on the wall of the cannula only at certain points.

According to an even further alternative, the electric lines may be arranged to extend helically around the cannula. The effect is substantially the same as with the meander-like extending electric lines.

For instance, the cannula may include a reinforcing structure, such as ring-shaped reinforcing elements or reinforcing windings which extend helically along the wall of the cannula, and the helically extending electric lines may be arranged in a specific relation to those reinforcing windings or reinforcing elements.

In a first embodiment including helically extending electric lines, an angular orientation of the helically extending electric lines relative to the longitudinal axis of the cannula may be different to an angular orientation of the one or more helically extending reinforcing windings so that the reinforcing windings and the electric lines overlap. The angular orientations preferably differ by at least 5°, preferably at least 10°, to avoid that the electric lines slip in between the reinforcing windings. More preferably, the angular orientation of the electric lines is opposite to the angular orientation of the reinforcing windings, for instance + 5° on the one hand and - 10° on the other hand or + 20° on the one hand and - 20° on the other hand.

In a second embodiment, the helically extending electric lines may be placed between the one or more helically extending reinforcing windings so as to be nested between them. Where a plurality of reinforcing windings is present, these windings may be nested within each other, and where a plurality of helically extending electric lines are provided, these may preferably be arranged such that exactly one of the electric lines is placed between two reinforcing windings. The arrangement may be such that more than one, e.g. two or three, of the helically extending reinforcing windings are present between neighboring electric lines. Preferably, between two and nine helically extending reinforcing windings may be present. In the case that there are three electric lines and nine reinforcing windings, there may be three reinforcing windings arranged between the individual electric lines. As a result, the more reinforcing windings are present, the shorter is the angle between the longitudinal axis of the cannula and the orientation of the reinforcing windings, which means that - depending on the width of the reinforcing windings - the number of helical revolutions of the electric lines around the cannula decreases with the number of helically extending reinforcing windings. Accordingly, the angulation of the helically extending electric lines may be controlled by the number and width of the reinforcing windings. It is therefore preferred to make the helically extending reinforcing windings from a flat wire of predetermined width.

Most preferably, the reinforcing windings are made of a shape memory alloy, such as nitinol. It is preferred to arrange an electrically insulating layer between the helically extending reinforcing windings and the helically extending electric lines such that the reinforcing windings are arranged above the insulating layer and the electric lines are arranged below the insulating layer, or vice versa. This way, the electric lines are insulated against each other and, more particularly, against the reinforcing windings.

In an even further alternative embodiment, one or more of the electric lines may be formed by respective ones of the helically extending reinforcing windings. Again, it is preferred to arrange an electrically insulating layer between the helically extending reinforcing windings in such a case in a manner that neighboring reinforcing windings are arranged in alternating fashion above and below the insulating layer in order to prevent that the reinforcing windings may contact each other and create a short circuit.

In all of the embodiments and alternatives described above, it is preferred to arrange the electric lines inside the wall of the cannula in order to protect them. Preferably, the wall of the cannula comprises a first foil and a second foil, the second foil being bonded to and covering the first foil. At least one reinforcement member, such as the aforementioned reinforcing windings, may be arranged between the first and second foil, preferably together with the electric lines. For instance, the first and second foils may be made of polyurethane.

One or more electrical connectors, such as a printed circuit board or simply an insulating coating, may be provided at the proximal end and/or at the distal end of the cannula. For instance, an electrical connector at or distally to the distal end of the cannula may be provided and used to connect each of the motor cables with a stranded wire or single wire. The stranded or single wires thus form the electric lines and are laid along the cannula in one of the ways as described above. Stranded wires are preferred over single wires because they are electrically conductive even when one strand in the stranded wire breaks. Preferably, the stranded wires of the electric cable arriving through the supply line may form the electric lines which extend across the cannula and may then be electrically connected to the individual motor cables of the drive section via the electrical connector provided at the distal end of the cannula. More preferably, the stranded wires may be different to the stranded wires of the electric cable arriving from the supply line and may be adapted to the specific requirements for the purpose of extending across the cannula. In this case, electrical connectors are provided at both ends of the cannula in order to connect the specific stranded wires, or alternatively specific single wires, with one end to the motor cables and with another end to the stranded wires of the electric cable of the supply line. Finally, one or more electrical connectors may be provided solely at the proximal end of the cannula, namely in the case where the motor cables extend all the way across the cannula, such as in the above described embodiment where the motor cables are guided through one or more bendable tubes.

As mentioned, one or more anchors may be provided at the distal end of the pumping device, namely preferably on the drive section, in order to anchor the pumping device in e.g. an aortic wall of the patient so as to stably fix the position of the pumping device inside the patient's heart.

Most preferably, the cannula is configured not to buckle when bent along its longitudinal axis by up to 180°. For instance, when the cannula is supposed to extend from the left atrium to the aorta and thereby bridge the left ventricle, the predetermined curvature of the cannula is not far from 180°, whereas the cannula has to assume an almost longitudinally stretched configuration when it is maneuvered through the patient's veins.

Generally, the blood pump may be configured to be used in a long-term application, wherein the blood pump is preferably configured to be operated in a patient for at least four weeks, more preferably at least six months.

### BRIEF DESCRIPTION OF THE DRAWINGS

The foregoing summary, as well as the following detailed description of preferred embodiments, will be better understood when read in conjunction with the appended drawings. For the purpose of illustrating the present disclosure, reference is made to the drawings. The scope of the disclosure is not limited, however, to the specific embodiments disclosed in the drawings. In the drawings:
Figs. 1 to 11 show the placement of an intravascular blood pump through the venous vasculature according to a first placement principle;
Figs. 12 to 15 show the introduction of an intravascular blood pump through the venous vasculature according to a second placement principle;
Fig. 16 shows the intravascular blood pump introduced through the apex of the human heart into the left ventricle according to a third placement principle;
Fig. 17 shows an intravascular blood pump according to a first embodiment;
Fig. 18 shows an intravascular blood pump according to a second embodiment;
Fig. 19A to 19C show in intravascular blood pump according to a third and fourth embodiment;
Fig. 20 shows an intravascular blood pump according to a fifth embodiment;
Fig. 21A to 21B show an intravascular blood pump according to a sixth and seventh embodiment;
Fig. 22A to 22C show an intravascular blood pump according to an eighth embodiment;
Fig. 23A to 23D show an intravascular blood pump according to a ninth and tenth embodiment; and
Figs. 24 to 30 show different anchoring structures provided at the distal end of the pumping device along with suitable grippers of connecting catheters for attaching to the anchoring structures.

### DETAILED DESCRIPTION

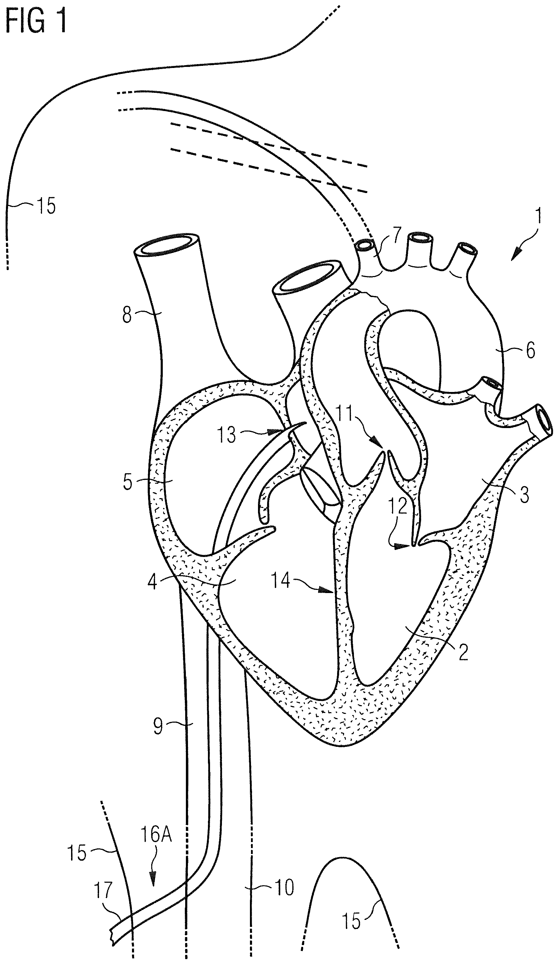

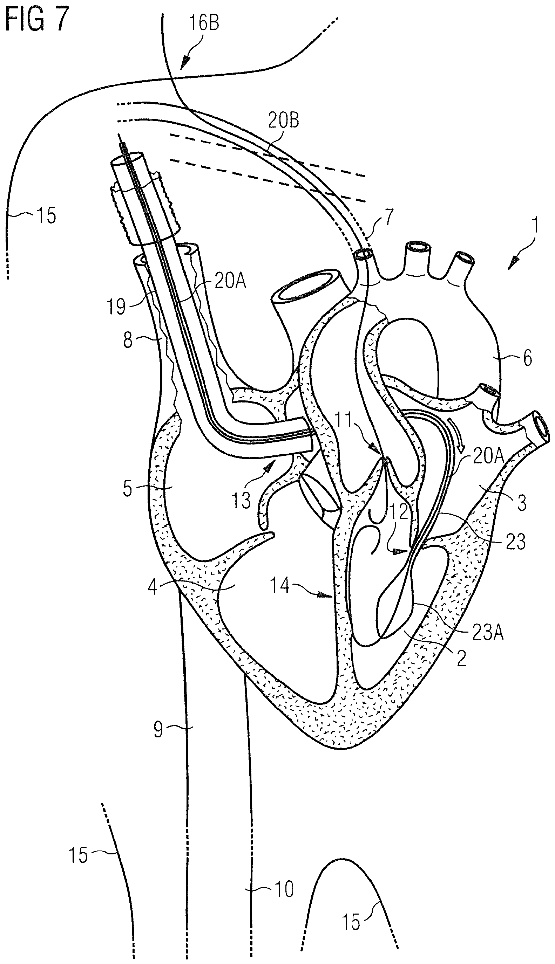

Figs. 1 to 16 show various principles for placing an intravascular blood pump in a patient's heart. Fig. 1 shows a cross section of the human heart 1 exposing the left ventricle 2, left atrium 3, right ventricle 4, right atrium 5 and the ascending part of the aorta 6. Relevant for the further understanding are the position of the subclavian artery 7, superior vena cava 8, inferior vena cava 9 and femoral vein 10 as well as the position of the aortic valve 11, mitral valve 12, atrial septum 13 and ventricular septum 14. Designated with the reference numeral 15 is the skin of the patient.

The arterial vessel system or arterial vasculature comprises the left atrium 3, the left ventricle 2, the aorta 6 and the subclavian artery 7, whereas the venous vessel system or venous vasculature comprises the femoral vein 10, the inferior vena cava 9, the superior vena cava 8, the right atrium 5, and the right ventricle 4. The atrium septum 13 separates the left and right atriums 3 and 5, and the ventricular septum 14 separates the left and right ventricles 2 and 4.

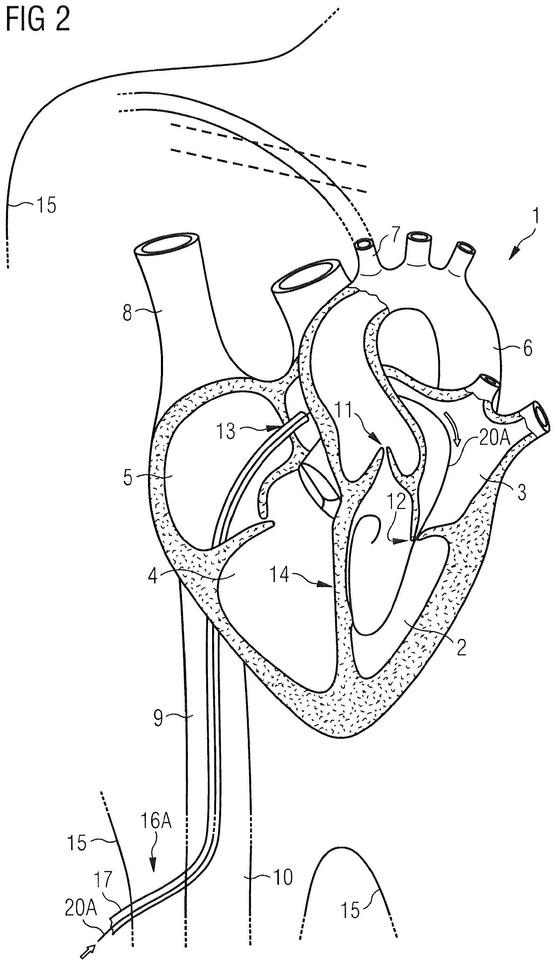

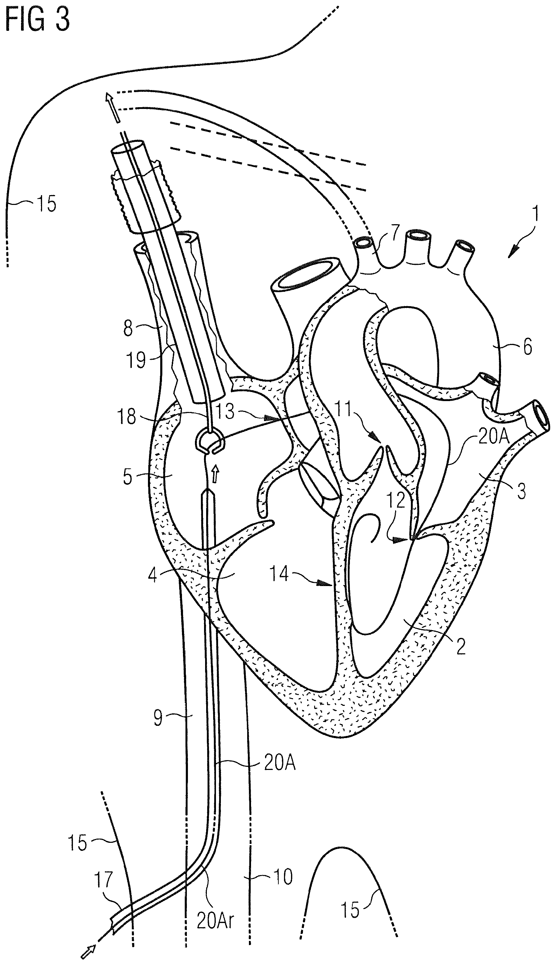

According to the first placement principle, a venous vessel is accessed by a percutaneous access 16A according to the Seldinger technique. Preferably the femoral vein 10 is accessed, but alternatively a subclavian vein (not shown, leading to the superior vena cava 8) may be accessed instead of the femoral vein. However, for the process as described hereinafter, namely to place in a first step a guide wire through the venous system and the atrial septum into the left part of the heart, it is advantageous to access the femoral vein 10 and insert a transseptal sheath through the lower percutaneous access 16A towards the atrial septum 1 3 and perforate the atrial septum 1 3 using the tip end of the transseptal sheath 17 as shown in Fig. 1. Alternatively, a separate needle may be used to perforate the septum. Next, as shown in Fig. 2, a first guide wire 20A is advanced through the transseptal sheath 17 into the left part of the heart up into the left ventricle 2. It is relatively easy to advance a transseptal sheath and guide wire through the atrial septum into the left part of the heart, in particular up into the left ventricle, when coming from the vena cava inferior, namely because of the natural curvature within the human ventricular system, including the curvature in the heart. However, the further procedural steps of the first placement principle are easier to perform coming from the vena cava superior. Therefore, as shown in Fig. 3, a snare catheter 18 is advanced through a sheath 19 placed in the vena cava superior 8 to pull up the first guide wire 20A. The sheath 19 may later be used for introducing the intravascular blood pump into the vasculature. The sheath 19 and snare catheter 18 may be inserted through an upper percutaneous access into the vein above the vena cava superior, such as into the subclavian vein or axillary vein, and advanced through the vena cava superior 8 towards the first guide wire 20A in the vena cava inferior 9, where it is used to catch the first guide wire 20A with appropriate grips provided at the end of the snare catheter 18. When the first guide wire 20A is catched using the snare catheter 18, it is subsequently pulled into the sheath 19 and out of the upper percutaneous access (not shown) until the rear end 20Ar of the first guide wire 20A appears outside the patient's body, as generally indicated by arrows in Fig. 3. For this purpose, the transseptal sheath 17 is withdrawn a short distance so that the snare catheter 18 can grip the first guide wire 20A in the right atrium. After the rear end 20Ar has been pulled out through the upper percutaneous access (not shown), the transseptal sheath 17 may be entirely withdrawn from the patient's body.

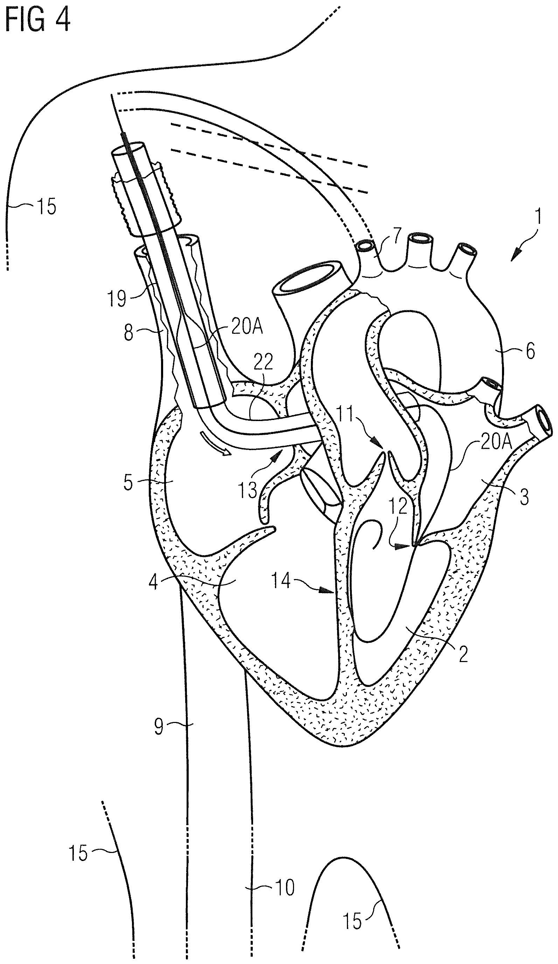

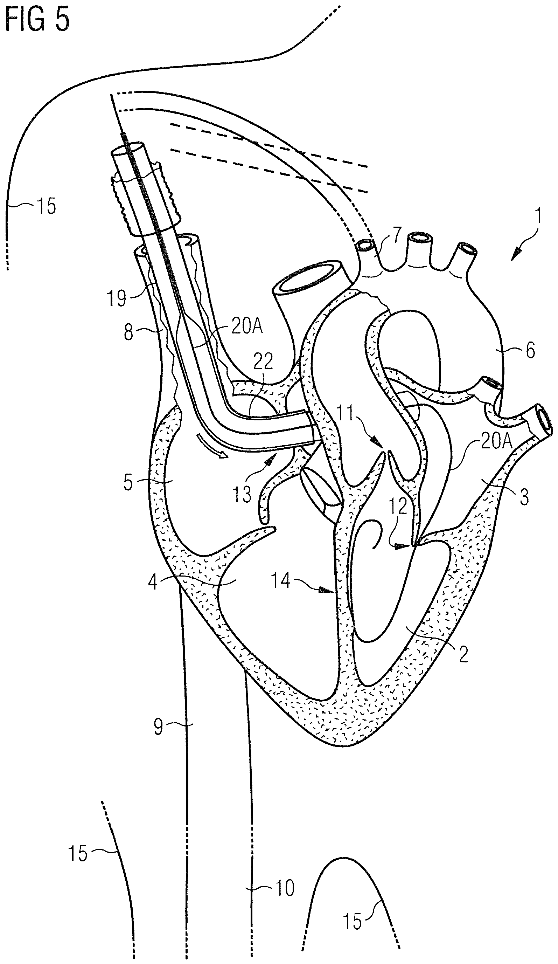

Next, as shown in Fig. 4, a balloon catheter 22, which is a PTA balloon in the embodiment shown but which may be any kind of balloon, is guided along the guide wire 20A through the sheath 19 and further through the atrial septum and is inflated so as to expand and, thereby, expand the perfusion through the atrial septum 13. Once the perfusion through the atrial septum 13 has been increased to an appropriate size, the sheath 19 is advanced over the PTA balloon 22 so as to extend into the left atrium 3, as shown in Fig. 5.

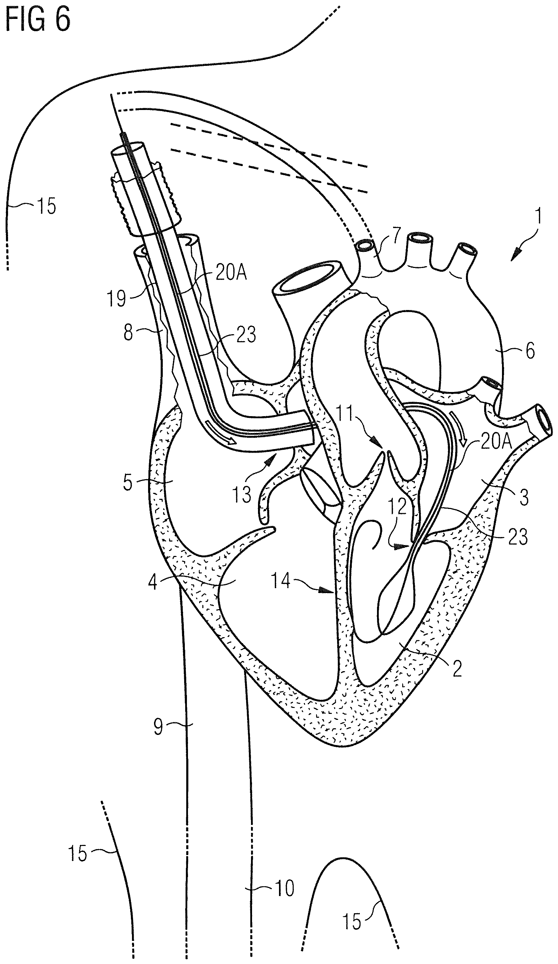

Thereafter, the PTA balloon 22 may be withdrawn and a maneuverable catheter 23, which is a Swan-Ganz catheter in the embodiment shown but which may be any suitable guide catheter, such as a pigtail catheter, is inserted instead. The Swan-Ganz catheter 23 is guided along the first guide wire 20A until it reaches into the left ventricle 2, as shown in Fig. 6. Next, a second guide wire 20B is introduced, preferably through the subclavian artery 7, into the left ventricle 2 in order to aid the further procedure of guiding the first guide wire 20A to an arterial percutaneous access 16B. More specifically, the second guide wire is visible in X-ray imaging. Then, the balloon 23A at a distal end of the Swan-Ganz catheter 23 is inflated so that natural blood flow supports further movement of the Swan-Ganz catheter 23 along with the first guide wire 20A through the vasculature up into and through the subclavian artery 7. The provision of the second guide wire 20B is optional. Instead of the second guide wire 20B, a connecting catheter with a grip may be used to attach to the front end 20Af of the first guide wire 20A. However, even when a connecting catheter is used to catch the front end 20Af of the first guide wire 20A, the Swan-Ganz catheter may nevertheless be used to further advance the first guide wire 20A by pushing because solely pulling the guide wire through the vasculature of the patient may harm the patient's vessel tissue.

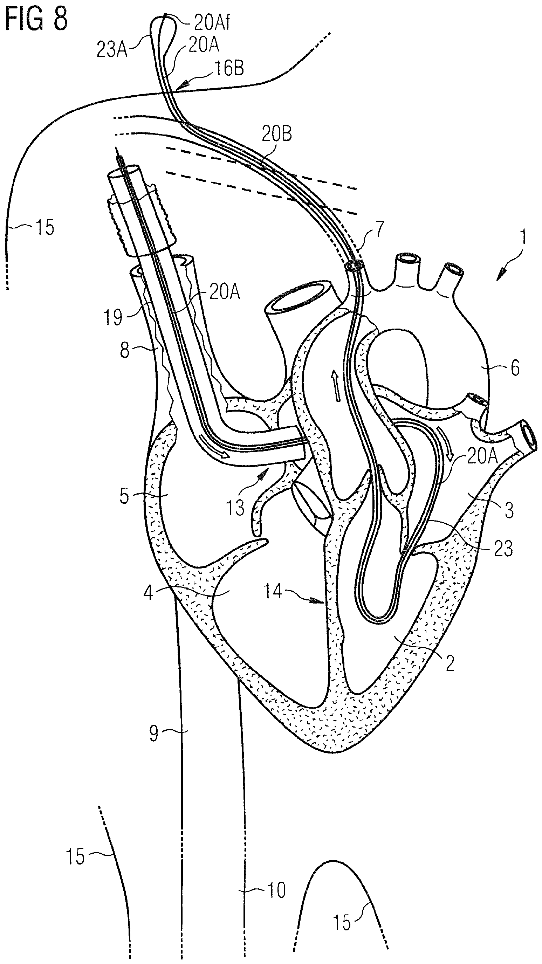

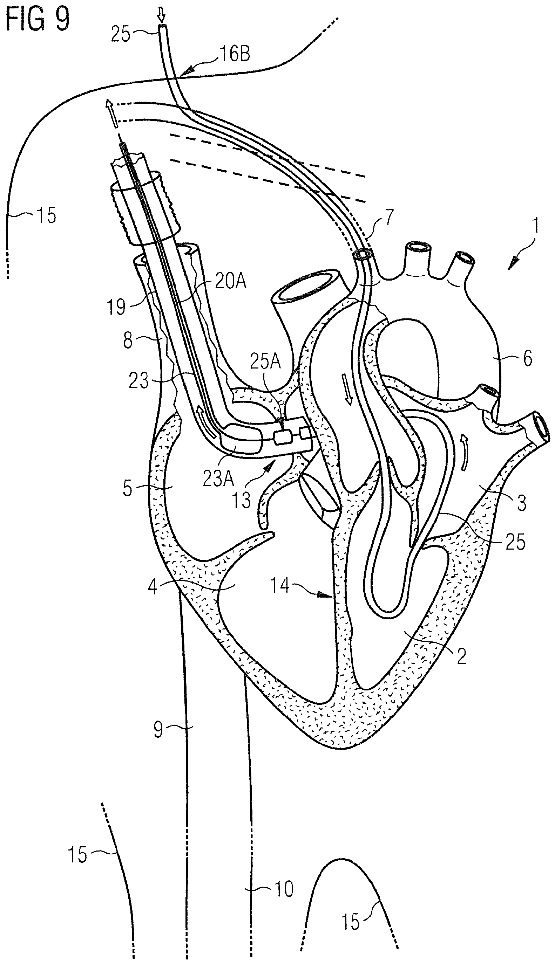

Once the front end 20Af of the first guide wire 20A and the balloon 23A of the Swan-Ganz catheter become visible to the surgeon, as shown in Fig. 8, a connecting catheter 25 can be securely fastened to the front end 20Af of the first guide wire 20A. The connecting catheter 25 may be the same as the snare catheter 18. Then, as shown in Fig. 9, the coupling catheter 25 securely attached to the first guide wire 20A is guided in the opposite direction from the arterial percutaneous access 16B towards the upper percutaneous access on the venous side (not shown). While the connecting catheter 25 is advanced, the first guide wire 20A is retracted and, at the same time, also the Swan-Ganz catheter 23 is retracted. Fig. 9 shows such movement at a moment when the - meanwhile deflated - balloon 23A of the Swan-Ganz catheter 23 has entered the sheath 19 in a rearward direction together with the front end 25A of the connecting catheter 25 attached to the front end 20Af of the first guide wire 20A.

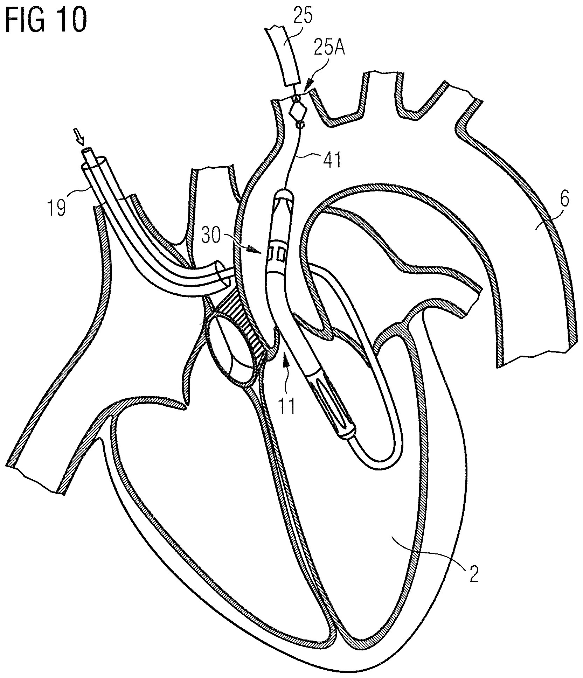

Next, when the front end 25A of the connecting catheter 25 is reaching out of the patient's body through the sheath 19, it is attached to the distal end of the pumping device 30 and used to guide the pumping device in a reverse direction through the sheath 19 into the right part of the heart until the pumping device 30 bridges the aortic valve 11, as shown in Fig. 10. In the embodiment shown here, a soft pigtail extension is provided at the end of the pumping device 30, and the front end 25A of the connecting catheter 25 is attached to a loop at the distal end of the pigtail extension.

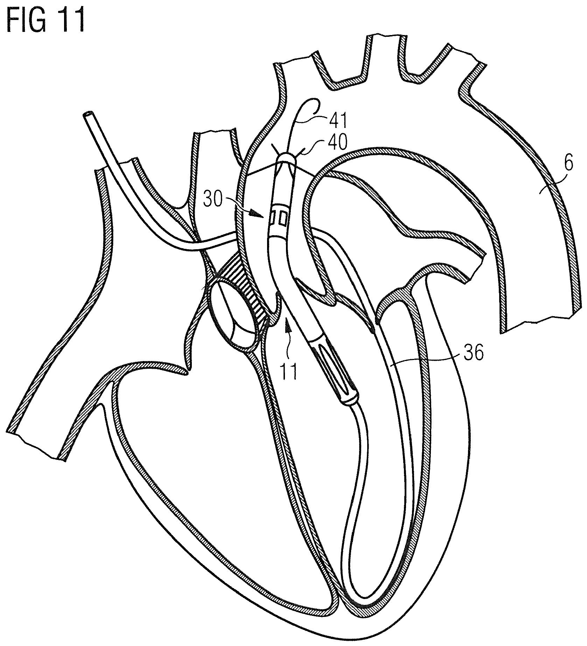

When the position as shown in Fig. 10 has been reached, the connecting catheter 25 is disconnected and removed, whereas the supply catheter 36 of the blood pump, which extends through the heart into the left ventricle and forms a loop therein, is further advanced so as come to lie against the heart wall of the left ventricle, as shown in Fig. 11. In this configuration, the supply catheter 36 supports the position of the pumping device 30 and prevents it from moving into the left ventricle during its operation. Optionally, spikes 40 may be provided and extended from the pumping device 30 so as to anchor the pumping device 30 in the aorta 6, as likewise shown in Fig. 11.

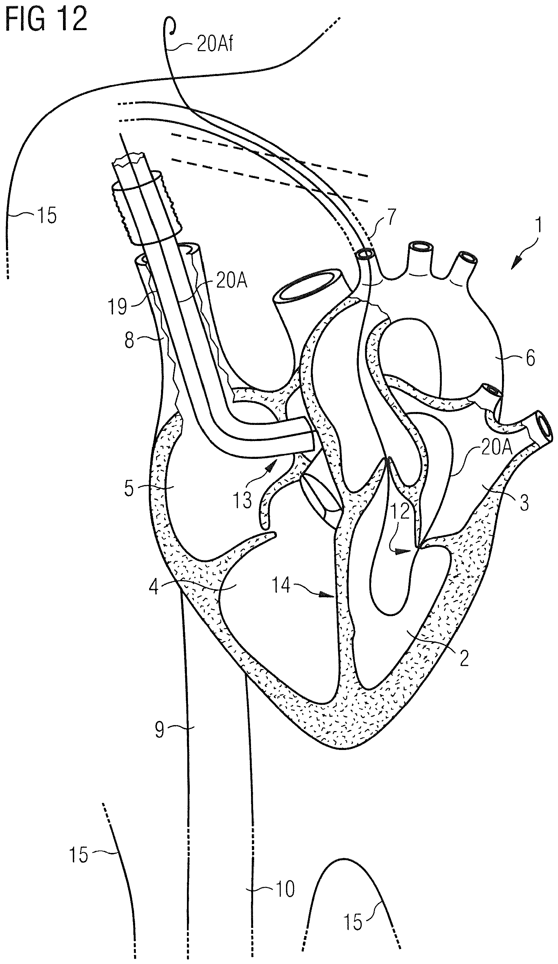

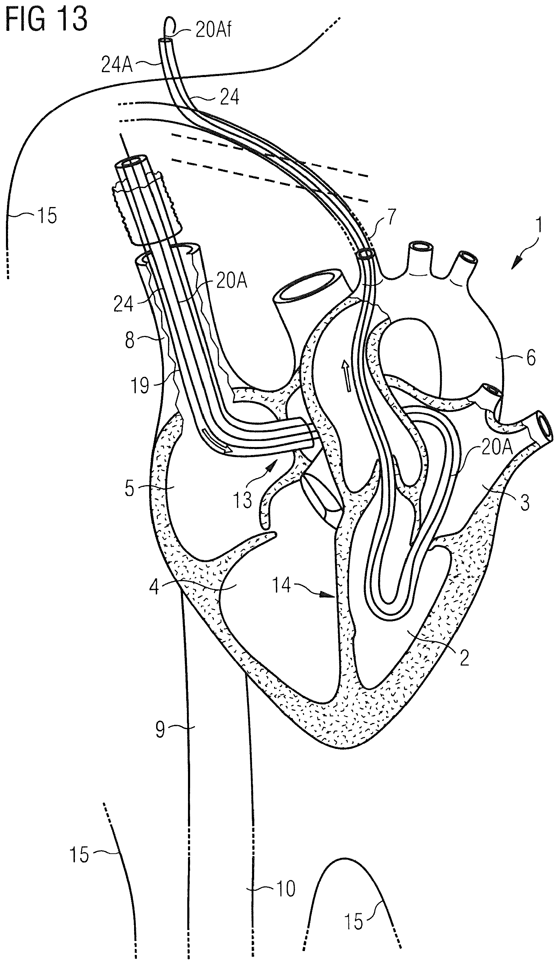

The above described placement method according to the first principle in which the first guide wire 20A extends all the way through the patient's vasculature and out of the patient's body both on a venous side and on an arterial side, can optionally be altered as described hereinafter. According to the second option of the first placement principle, all steps as described hereinabove in relation to Figs. 1 to 8 may be performed identically. However, once the front end 20Af of the first guide wire 20A is reaching out of the arterial side of the patient's vasculature, the Swan-Ganz catheter 23 may be completely withdrawn, as shown in Fig. 12. Next, a tube 24 is inserted into the sheath 19 and guided further along the guide wire 20A until it likewise reaches out of the patient's body with its front end 24A, as shown in Fig. 13. Alternatively, the tube 24 may be guided through the vasculature in the opposite direction.

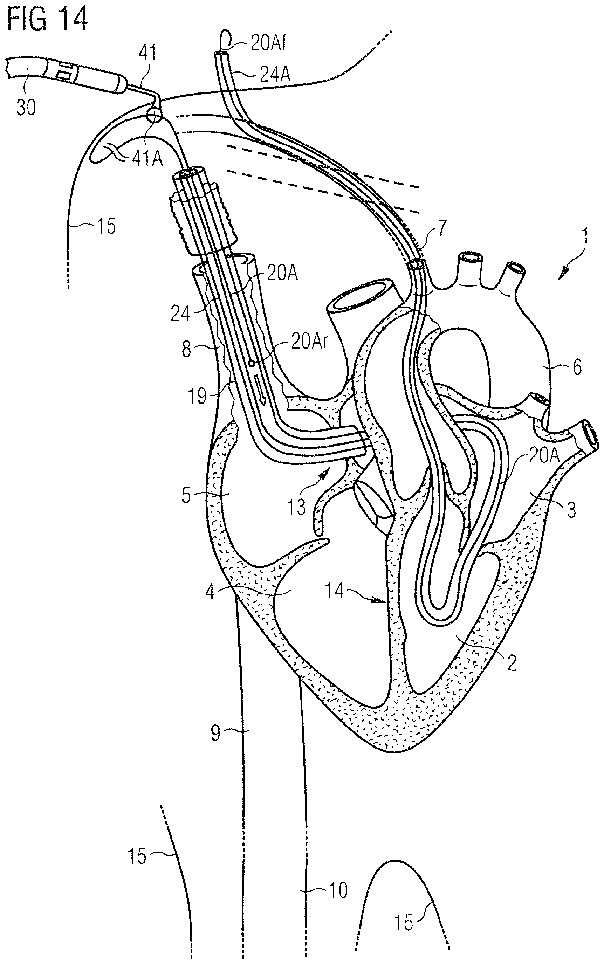

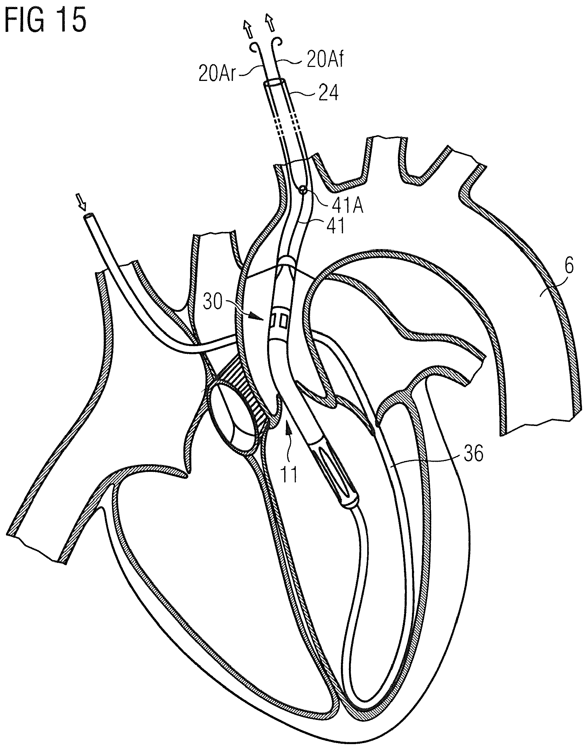

Next, as shown in Fig. 14, the rear end 20Ar is fed through a loop 41A at the end of the pigtail 41 of the pumping device 30 and further into the sheath 19, as shown in Fig. 14, and further all the way through the tube 24 until it reaches out of the front end 24A of the tube 24 outside the patient's body. Thus, both ends 20Af and 20Ar will extend out of the tube 24 on an arterial side of the patient. Next, as shown in Fig. 15, the two ends 20Af and 20Ar of the first guide wire 20A are being pulled along with the tube 24 while at the same time the pumping device 30 is advanced until it reaches the desired position within the patient's heart. The diameter of the tube 24 is chosen such that the pumping device 30 contacts against the tube 24 with its distal end while it is being advanced into the vasculature. Accordingly, when the pumping device 30 has reached the position as shown in Fig. 15, the first guide wire 20A may be unfed from the loop 41A of the pigtail extension 41 through the arterial percutaneous access 16B (not shown here), and also the tube 24 may be removed from the patient's body through the same arterial percutaneous access 16B. Finally, when the optional spikes 40 are extended into the wall of the aorta 6, the same final placement has been achieved as shown in Fig. 11.

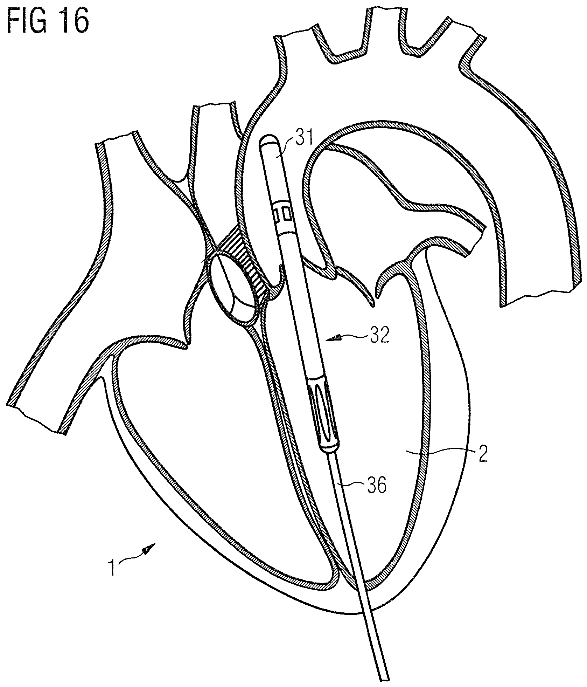

A placement method according to a second placement principle is shown in Fig.16. Here, the patient's heart 1 is accessed through the patient's thoracic cavity, and a puncture is created through the apical wall of the heart to access the left ventricle 2. In this case, the pump section 32 is arranged again between the drive section 31 and the supply line 36 or supply catheter. An intravascular blood pump as will be described herein after is particularly suitable to be placed inside a patient's heart according to this third placement principle. More specifically, the pumping device 32 is advanced through the puncture in the apical wall such that the blood flow inlet 33 of the pump section 30 is disposed in the left ventricle 2 and the blood flow outlet 34 of the pump section 32 is disposed in the patient's aorta 6. The supply line 36 extends through the puncture in the apical wall and out of the patient's body, and the drive section 31 is disposed in the aorta. Accordingly, all the advantages that have been described above in relation to the first placement principle as shown in Figs. 1 through 5 are likewise achieved with this third placement principle.

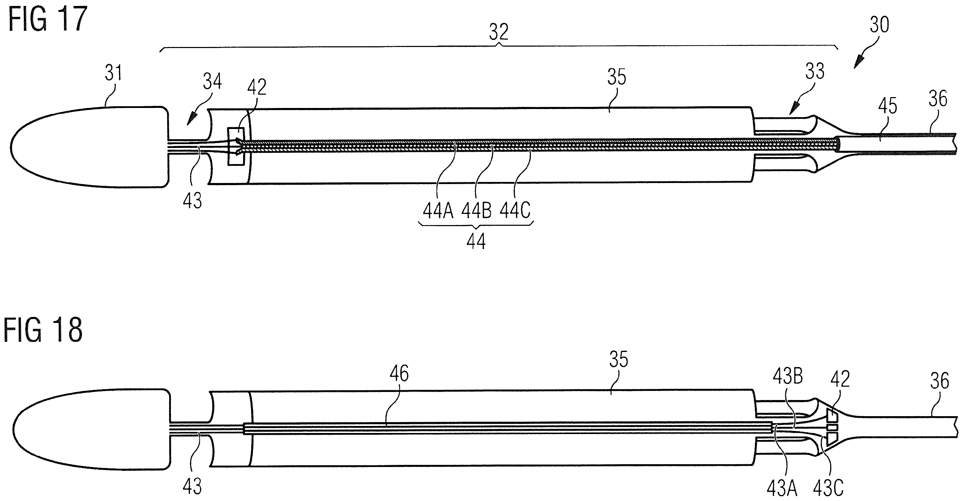

Hereinafter, a preferred intravascular blood pump particularly useful for the first and second placement principles will be described in connection with Figs. 17 to 23 which show 9 different embodiments of an intravascular blood pump.

The blood pump as shown in Figs. 17 to 23 each include a pump section 30 and a supply line 36, which is exemplified in these embodiments as a supply catheter, attached in a usual way to the proximal end of the pumping device 30. The pumping device includes a drive section 31 and a pump section 32, wherein the pump section 32 includes a blood flow inlet 33 having various inlet ports and a blood flow outlet 34 having various outlet ports and a cannula 35 extending between the blood flow inlet and blood flow outlet, the cannula being bendable. The pump section 32 further includes an impeller, not shown here, which is driven by a motor arranged in the drive section 31, the impeller being arranged in the area of the blood flow outlet 34 and having an axial as well as a radial component so as to propel the blood diagonally out of the blood flow outlet 34. Importantly, in all embodiments the pump section 32 is arranged between the supply line 36 and the drive section 31.

In the first embodiment shown in Fig.17, an electrical connector 42 is provided at a distal end of the cannula 35 next to the blood flow outlet 34. The electrical connector 42 is a printed circuit board to which the individual motor cables are attached, in the example shown three cables, namely an outer conductor, a neutral or zero conductor and a protective conductor, jointly referred to as motor cables 43. Also attached to the distal electrical connector 42 are the corresponding electric lines 44A, 44B and 44C, which are jointly referred to as electric lines 44. The electrical connector 42 is covered with an insulating material, such as polyurethane. In the embodiment shown, the electric lines 44 are extensions of the corresponding conductors in the electric cable 45 arriving at the pumping device 30 through the supply catheter 36. As such, the electric lines 44 are typically stranded wires. The electric lines 44 extend strictly longitudinal along the longitudinal axis of the cannula 35, namely along a neutral bending plane of the cannula 35 so that rupture of the electric lines 44 upon bending of the cannula 35 is prevented. In addition, the electric lines 44 may be chosen with appropriate stretch ability. If conductors of the electrical cable 45 are not sufficiently stretchable, they may be replaced by more stretchable stranded wires to form the electric lines 44 extending across the cannula 35. For this, one or more additional electrical connectors are needed at a location proximal of the blood flow inlet 33 (now shown here).

Fig. 18 shows a second embodiment which differs from the first embodiment in that the motor cables 43 form the electric lines 44 and extend along the length of the cannula 35 to the proximal end of the pumping device 30, where each of the individual motor cables 43A, 43B, 43C is soldered to corresponding printed circuit boards. Two measures are taken here to avoid rupture of the motor cables 43, which measures may be taken individually or, as here, jointly. First, the motor cables 43 are guided through tubes 46, in the embodiment shown one tube 46 per motor cable 43. The tubes 46 are bendable, preferably having a low bending stiffness in order not to obstruct the maneuverability of the pumping device. Further preferably, the tubes 46 are elastically bendable, i.e. they automatically return to their original form once the bending force is sufficiently reduced. Second, the motor cables 43 are arranged with slack, the slack being sufficient for the motor cables 43 to stretch without rupturing when the bendable tubes 46 are bent together with the cannula 35.

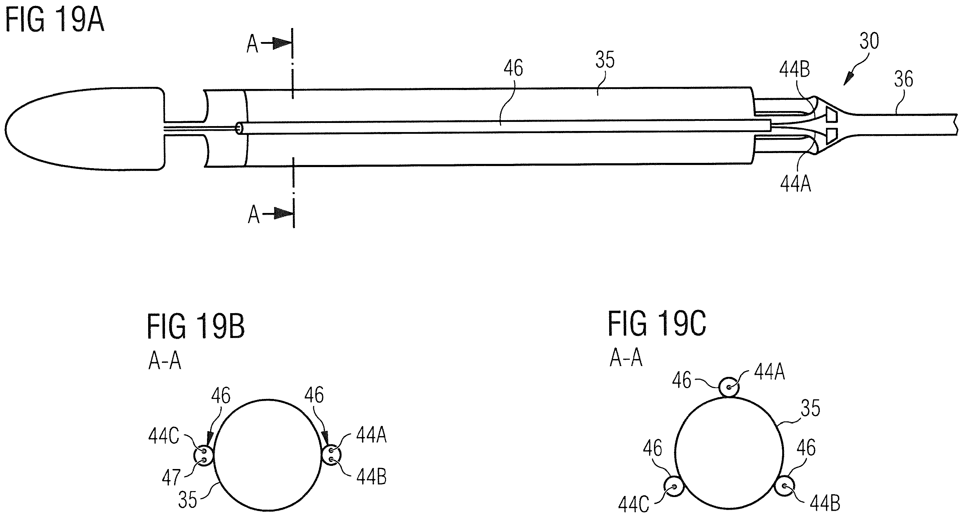

Figs. 19A and 19B show a third embodiment which differs from the second embodiment in that two bendable tubes 46 are arranged on opposite sides of the cannula 35 so as to create a neutral bending plane within the cannula 35. While the pumping device 30 as shown in Figs. 17 to 23 is shown in a stretched configuration, it assumes a curved configuration in a relaxed state, and the bendable tubes 46 in the embodiment shown in Figs. 19A and 19B are preferably arranged along the predetermined central plane of curvature of the cannula 35. In this case, there is no particular need to provide the motor cables 43 with much slack. As can be seen in Fig.19B, a first and a second supply line 44A, 44B may be arranged in one of the two bendable tubes 46 and the third supply line 44C together with a pressure line 47, such as an optical fiber or glass fiber strand, may be arranged in the other one of the two bendable tubes 46.

In a fourth embodiment shown in Fig.19C, the bendable tubes 46 are longitudinally arranged along the cannula 35 with an equal angular distance. In the example shown, the angular distance between the bendable tubes 46 is 120°. Where a fourth bendable tube 46 is provided e.g. for the pressure line 47, the angular distance would be 90°.

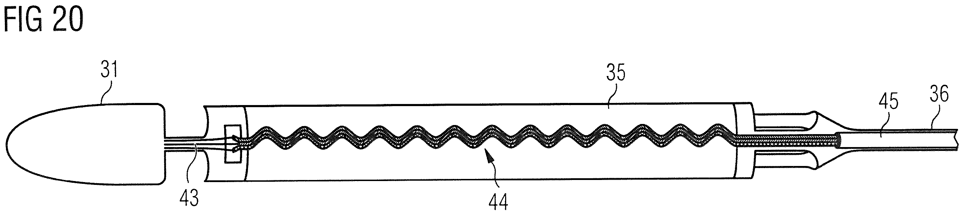

A fifth embodiment is shown in Fig. 20. Here, the electric lines 44 are not arranged in a strict axial direction but are disposed meander-like along the longitudinal axis of the cannula 35. In particular in the case where the electric lines 44 are each comprised of a stranded wire, here represented as an extension of the conductors of the electric cable 45 arriving through the supply catheter 36, rupture of the electric lines 44 upon bending of the cannula 35 is effectively prevented. Alternatively, the meander-like arranged electric lines 44 may be formed by single wires such as the individual motor cables 43. The flexibility of the meander-like arranged electric lines 44 can be enhanced further by fixing the electric lines 44 only at certain spaced-part points to the wall of the cannula 35.

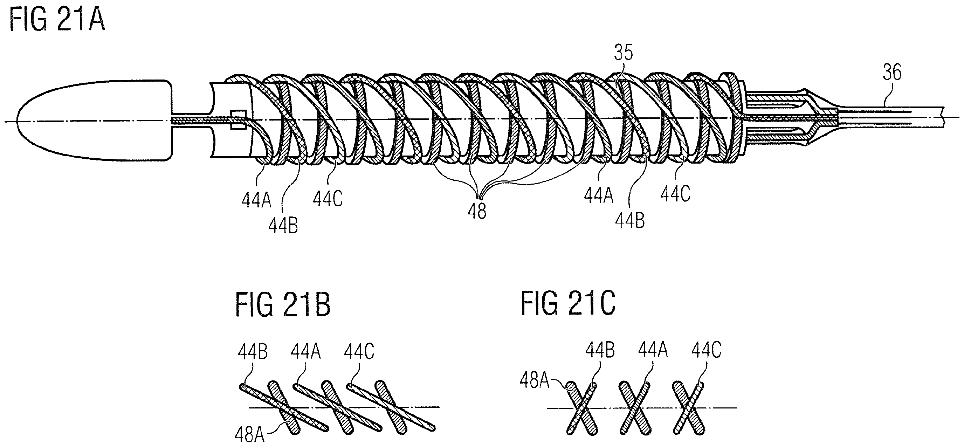

Fig. 21A shows a sixth embodiment of an intravascular blood pump wherein the cannula 35 includes reinforcing elements 48 to increase the stability of the cannula 35. The reinforcing elements 48 may be ring-shaped, as shown in Fig. 21A, or may be formed by one or more helical winding as shown in Figs. 21B and 21C. The same or similar reinforcements may likewise be provided in the cannula 35 of the above described embodiments. Importantly for this embodiment is the fact that the electric lines 44A to 44C extend helically around the cannula 35 along the longitudinal axis of the cannula 35. An angular orientation of the helically extending electric lines 44A to 44C relative to the longitudinal axis of the cannula 35 is different to the angular orientation of the ring-shaped reinforcing elements 48. In the specific embodiment shown in Fig. 21A, the reinforcing element 48 is formed as a helically extending reinforcing winding 48A, and the angular orientation of the helically extending electric lines 44A to 44C is different to the angular orientation of the helically extending reinforcing winding 48A so that the reinforcing winding 48A and the electric lines 44 overlap. Preferably, the angular orientations differ by at least 5°, preferably at least 10°, in the example of Fig. 21B about 20°.

In a seventh embodiment as shown in Fig. 21C, the angular orientation of the helically extending electric lines 44 is opposite to the angular orientation of the helically extending reinforcing windings 48A, namely about +40° and -40° as compared to a circular orientation.

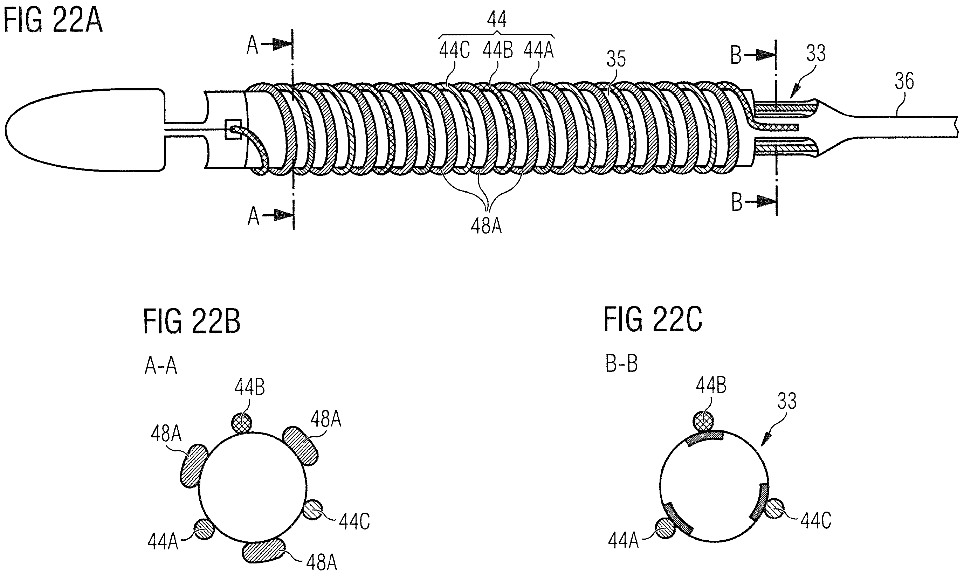

An eighth embodiment is shown in Figs. 22A to 22C. Here, the helically extending electric lines 44A to 44C are placed between the helically extending reinforcing windings 48A so that each of the electric lines 44 is nested between two of the electric lines 48A. As can be seen from the cross-sectional view as shown in Fig. 22B, altogether three of the helically extending reinforcing windings 48A are present, the reinforcing windings being made of a flat wire, preferable comprising a shape memory material such as nitinol. As can be seen in Fig. 22C, the blood flow inlet 33 has three inlet ports separated by respective three bridges along each of which one of the electric supply lines 44 is laid.

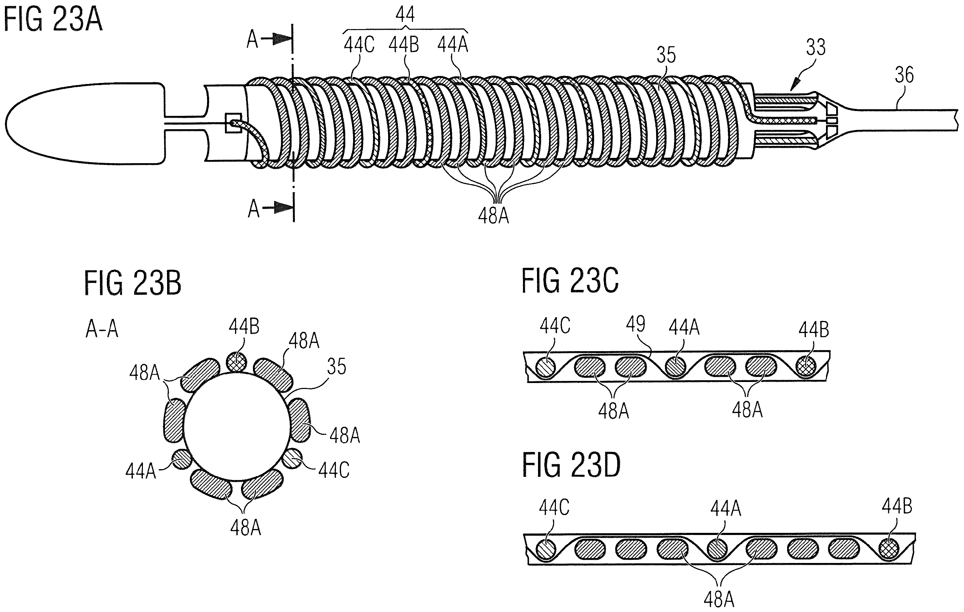

Fig. 23A to 23C shows a ninth embodiment with even more helically extending reinforcing windings 48A. Altogether, six reinforcing windings 48A are arranged along the cannula 35, and the electric lines 44A to 44C are individually placed between them so that always two reinforcing windings 48A are positioned between neighboring ones of the electrical lines 44.

Fig. 23C shows a cross-section through the wall of the cannula 35. An electrically insulating layer 49 is arranged between the reinforcing windings 48A and the electric lines 44A to 44C such that the reinforcing windings 48A are arranged below the insulating layer and the electric lines 44A to 44C are arranged above the insulating layer 49. In addition, it can be seen that the wall comprises two foils 50, 51 forming an outer layer and an inner layer of the wall of the cannula 35. The reinforcing member 48 or reinforcing windings 48A are positioned between these foils, where applicable along with the electric lines 44 as is the case in the embodiment shown in Figs. 23A to 23C.

A tenth embodiment is shown in Fig. 23D according to which three reinforcing windings 48A are positioned between neighboring electric lines 44. Thus, altogether nine reinforcing windings 48A are present in this embodiment.

Alternatively, the reinforcing windings 48A may form the electric lines 44 extending along the cannula 35, and the insulating layer 49 may be used to isolate them against each other.

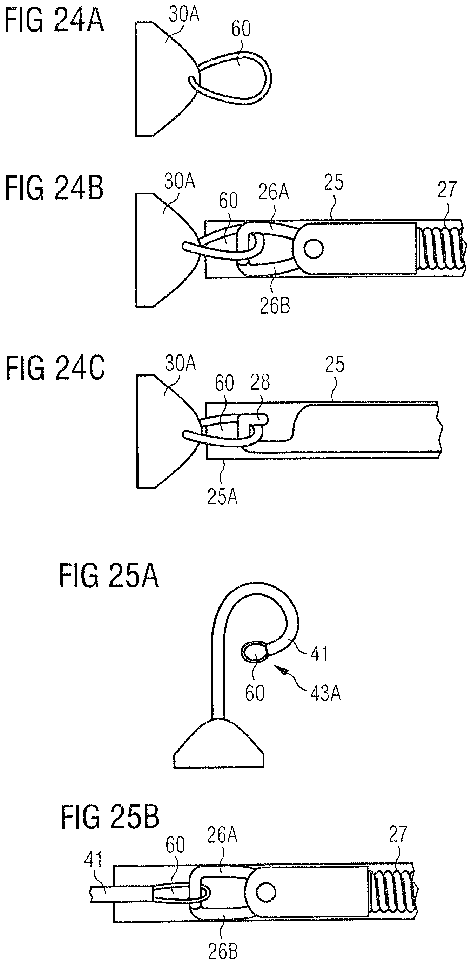

With respect to Figs. 24 to 30, preferred anchoring structures and preferred grips for attaching the connecting catheter 25 to corresponding anchoring structures of the pumping device 30 will be described. These anchoring structures and connecting catheters are useful in the above described placement methods according to the first placement principle in order to guide the blood pump from the venous vasculature through the atrial septum into the left ventricle and up into the aorta.

Accordingly, Fig. 24A shows a distal end 30A of a pumping device, which may be the housing of the drive section 31 of the pumping device 30. A loop 60 is provided on the distal end 30A and may consist of wires in a polymer sheath. Fig. 24B illustrates how a connecting catheter 25 is attached to the loop 60. Accordingly, pivotable grips 26A and 26B reach into the loop 60 and a spring element 27 urges the two grips 26A, 26B together so as to hold the grips 26A, 26B closed. Once the grips 26A, 26B are retracted into the connecting catheter 25, the grips 26A, 26B are prevented from opening. In this position, the pumping device 30 can safely be guided through the patient's vasculature. In Fig. 24C, the connecting catheter 25 includes a hook 28 rather than the grips 26A, 26B, which hooks into the loop 60 at the distal end 30A of the pumping device 30. Once the hook is retrieved in the front end 25A of the catheter 25, the loop 60 is safely prevented from loosening from the hook 28.

Figs. 25A and 25B show a similar embodiment which differs from the embodiment described above only in that the loop 60 is not provided on the distal end of the housing of the pumping device 30 but at the distal end 43A of a pigtail 41 which forms the distal end 30A of the pumping device.

Accordingly, an introducer set may comprise an intravascular blood pump having an anchoring structure at the distal end 30A of the pumping device 30 and a connecting catheter 25 adapted to connect into the anchoring structure.

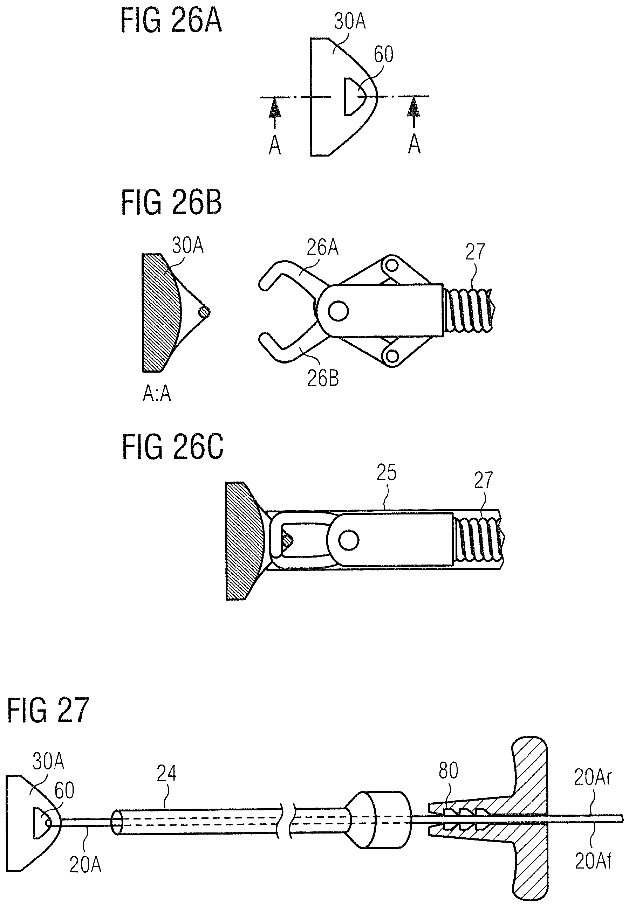

Figs. 26A to 26C show a different embodiment with a loop 60 at the distal end 30A of the pumping device 30. Here, the loop 60 is integrally formed in the housing of the pumping device 30.

While the embodiments shown in Figs. 24 to 26 relate to placement methods according to the first option of the first principle as described in relation to Figs. 9 to 11, Fig. 27 shows how the same anchoring structure can be used in context with the second option of placing a blood pump according to the first placement principle as described in relation to Figs. 12 to 15. Accordingly, the first guide wire 20A may be fed through the loop 60 and further with both ends 20Ar and 20Af through the sheath 24. The ends 20Ar and 20Af can then be gripped with the gripper 80 in order to guide the blood pump through the vasculature.

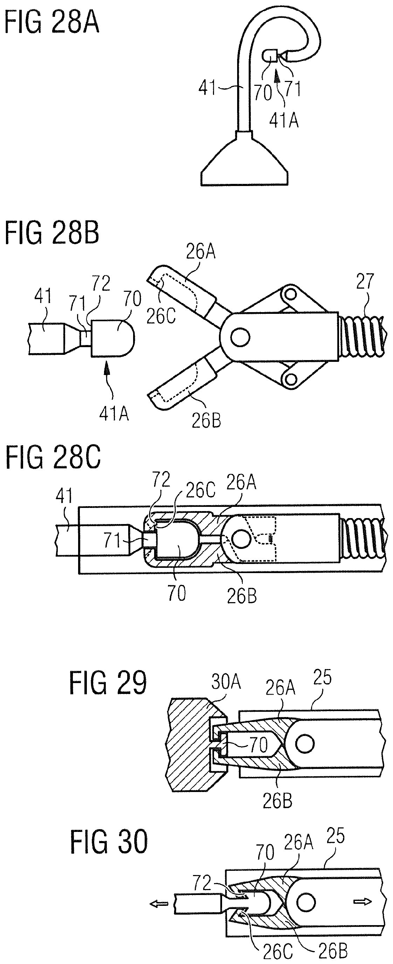

Fig. 28A shows a neck-head-structure at the end 41A of the pigtail 41, rather than a loop 60. Accordingly, the grips 26A, 26B of the connecting catheter 25 have a complementary surface so as to close around the neck-and-head structure. More specifically, the neck-head-structure includes a head 70 and a neck 71 and a connecting surface 72 between the head 70 and the neck 71. The connecting surface 72 is inclined by 90° relative to the longitudinal axis of the pigtail 41. A corresponding cooperating surface 26C on the grips 26A and 26B is likewise inclined by 90° relative to the longitudinal axis of the connecting catheter 25. Fig. 28C shows how the two surfaces 72 and 26C cooperate. Any forces acting between the two surfaces 72 and 26C are strictly axial which improves the connection.

Fig. 29 shows a similar design. However, here the head 70 is arranged in a recess at the distal end 30A of the pumping device 30 in order to prevent damage to the tissue in the patient's vasculature once the connecting catheter 25 has been detached.

Fig. 30 shows again a neck-and-head structure. However, in this embodiment the co-acting surfaces 26C and 72 are inclined by more than 90° relative to the respective longitudinal axis so that each surface forms an undercut with respect to the head and grips, respectively. This gripper structure is self-enhancing when the head 70 and the grippers 26A, 26B are moved in opposite axial directions, as indicated by the two arrows in Fig. 30.

Accordingly, a kit for accurately placing the intravascular blood pump in a patient according to the first placement principle may comprise the following tools:
- the transseptal sheath 17, which may include a puncture tip end or separate puncture needle and a dilator,
- the first guide wire 20A,
- the second guide wire 20B,
- the introducer sheath 19,
- the snare catheter 18,
- the balloon catheter 22 for extending the septum, such as the PTA balloon catheter,
- the maneuverable catheter 23, such as the Swan-Ganz catheter,
- either the connecting catheter 25 (according to the first option of the first placement principle as described in relation to Figs. 1 to 11) or the tube 24 (according to the second option of the first placement principle as described in relation to Figs. 1 to 8 and 12 to 15).

Preferred embodiments are specified in the following paragraphs:
1. An intravascular blood pump for percutaneous insertion into a patient's vasculature, the intravascular blood pump comprising a pumping device and a supply line,
   - wherein the pumping device comprises a pump section having a blood flow inlet, a blood flow outlet and an impeller rotatable about an axis of rotation for conveying blood from the blood flow inlet to the blood flow outlet, and a drive section connected to the pump section and adapted to drive the impeller,
   - wherein the supply line is adapted to supply the drive section at least with electric energy for driving the impeller, and
   - wherein the pump section is arranged along the axis of rotation between the drive section and the supply line.
2. The intravascular blood pump according to paragraph 1, comprising an electrically conductive connection which extends along the pump section and electrically connects the supply line with a motor of the drive section.
3. The intravascular blood pump according to paragraph 1 or 2, wherein the pump section comprises a cannula having a longitudinal axis and being bendable along the longitudinal axis.
4. The intravascular blood pump according to paragraph 3, wherein the blood flow outlet of the pump section is arranged at a distal end of the cannula.
5. The intravascular blood pump according to paragraph 3 or 4, wherein the electrically conductive connection comprises one or more electric lines which extend along a wall of the cannula and electrically connect the supply line with the motor of the drive section.
6. The intravascular blood pump according to paragraph 5, wherein the motor of the drive section includes a plurality of motor cables and wherein an electrical connector is provided at or in a distance distally to a distal end of the cannula, preferably proximal of the blood flow outlet, where each of the plurality of motor cables is respectively connected to one of the one or more electric lines, the electrical connector preferably being a printed circuit board, and the electrical connector preferably being covered with an insulating material, more preferably with a polymer coating, such as polyurethane.
7. The intravascular blood pump according to paragraph 6, wherein each one of the one or more electric lines is a stranded wire.
8. The intravascular blood pump according to paragraph 6, wherein each one of the one or more electric lines is a single wire.
9. The intravascular blood pump according to paragraph 5, wherein the motor of the drive section includes a plurality of motor cables which constitute the one or more electric lines extending along the cannula, and wherein an electrical connector is provided at or proximally of a proximal end of the cannula, preferably proximal of the blood flow inlet, where each of the plurality of motor cables is respectively connected to the supply line, the electrical connector preferably being a printed circuit board, and the electrical connector preferably being covered with an insulating material, more preferably with a polymer coating, such as polyurethane.
10. The intravascular blood pump according to any one of paragraphs 5 to 9, wherein the one or more electric lines extend along the wall of the cannula in a longitudinal direction parallel to the longitudinal axis of the cannula, preferably along a neutral bending plane of the cannula which is predetermined by a central plane of curvature of the cannula, and are stretchable in the longitudinal direction.
11. The intravascular blood pump according to any one of paragraphs 5 to 9, wherein the one or more electric lines extend along the wall of the cannula inside at least one bendable tube, which is preferably disposed along a neutral bending plane of the cannula which is predetermined by a central plane of curvature of the cannula.
12. The intravascular blood pump according to paragraph 11, wherein the one or more electric lines are laid with slack inside the at least one bendable tube.
13. The intravascular blood pump according to paragraph 11 or 12, wherein one bendable tube is provided for each one of the one or more electric lines.
14. The intravascular blood pump according to any one of paragraphs 11 to 13, wherein a plurality of the at least one bendable tube is arranged on opposite sides of the wall of the cannula so as to create a neutral bending plane within the cannula.
15. The intravascular blood pump according to paragraph 14, wherein the cannula has an even number of blood flow inlet ports, more preferably four blood flow inlet ports.
16. The intravascular blood pump according to any one of paragraphs 11 to 15, wherein the at least one bendable tube is made of a shape memory alloy.
17. The intravascular blood pump according to paragraph 16, wherein the shape memory alloy is nitinol.
18. The intravascular blood pump according to any one of paragraphs 5 to 9, wherein the one or more electric lines extend meander-like along the wall of the cannula.
19. The intravascular blood pump according to paragraph 18, wherein the one or more meander-like extending electric lines are arranged along a neutral bending plane of the cannula which is predetermined by a central plane of curvature of the cannula.
20. The intravascular blood pump according to any one of paragraphs 5 to 9, wherein the one or more electric lines extend helically along the wall of the cannula.
21. The intravascular blood pump according to paragraph 20, wherein the cannula includes one or more reinforcing windings which extend helically along the wall of the cannula and wherein an angular orientation of the one or more helically extending electric lines relative to the longitudinal axis of the cannula is different to an angular orientation of the one or more helically extending reinforcing windings so that the one or more reinforcing windings and the one or more electric lines overlap, wherein preferably the angular orientations differ by at least 5°, preferably at least 10°.
22. The intravascular blood pump according to paragraph 20 or 21, wherein the cannula includes one or more reinforcing windings which extend helically along the wall of the cannula and wherein an angular orientation of the one or more helically extending electric lines relative to the longitudinal axis of the cannula is opposite to an angular orientation of the one or more helically extending reinforcing windings so that the one or more reinforcing windings and one or more electric lines overlap.
23. The intravascular blood pump according to paragraph 20, wherein the cannula includes one or more reinforcing windings which extend helically along the wall of the cannula and wherein the one or more helically extending electric lines are placed between the one or more helically extending reinforcing windings.
24. The intravascular blood pump according to paragraph 23, wherein the one or more helically extending reinforcing windings include a plurality reinforcing windings arranged in parallel in an axial direction relative to the longitudinal axis of the cannula so as to be nested helically within each other, and wherein the one or more helically extending electric lines include a plurality of electric lines arranged such that exactly one of the plurality of electric lines is placed between two or more of the helically extending reinforcing windings.
25. The intravascular blood pump according to paragraph 24, wherein between two and nine of the reinforcing windings are present.
26. The intravascular blood pump according to any one of paragraphs 23 to 25, wherein one or two or three of the one or more helically extending reinforcing windings are arranged between each of the one or more helically extending electric lines.
27. The intravascular blood pump according to any one of paragraphs 23 to 26, wherein an electrically insulating layer is arranged between the helically extending reinforcing windings and the helically extending electric lines such that the reinforcing windings are arranged above the insulating layer and the electric lines are arranged below the insulating layer, or vice versa.
28. The intravascular blood pump according to paragraph 20, wherein the cannula includes one or more reinforcing windings, which extend helically along the wall of the cannula and wherein at least one of the electric lines is formed by a respective one of the reinforcing windings.
29. The intravascular blood pump according to paragraph 28, wherein an electrically insulating layer is arranged between the helically extending reinforcing windings such that neighboring reinforcing windings are arranged in alternating fashion above and below the insulating layer.
30. The intravascular blood pump according to any one of paragraphs 20 to 29, wherein the one or more helically extending reinforcing windings are made of a flat wire.
31. The intravascular blood pump according to any one of paragraphs 20 to 30, wherein the reinforcing windings are made of a shape memory alloy.
32. The intravascular blood pump according to any one of paragraphs 5 to 31, wherein the one or more electric lines are arranged inside the wall of the cannula.
33. The intravascular blood pump according to any one of paragraphs 5 to 32, wherein the wall of the cannula comprises a first foil and a second foil bonded to and covering the first foil, wherein at least one reinforcing member is arranged between the first and the second foils, preferably together with the one or more electric lines.
34. The intravascular blood pump according to paragraph 33, wherein the first and second foils are made of polyurethane.
35. The intravascular blood pump according to any one of paragraphs 3 to 34, wherein the cannula is configured not to buckle when bent along its longitudinal axis by up to 180°.
36. The intravascular blood pump according to any one of paragraphs 1 to 35, wherein the impeller is a axially-radially, diagonally or centrifugally delivering impeller.
37. The intravascular blood pump according to any one of paragraphs 1 to 36, wherein the supply line is in the form of a supply catheter which preferably further includes at least a pressure sensor line.
38. The intravascular blood pump according to any of claims 1 to 37, comprising at least one anchor adapted to anchor the intravascular blood pump in a wall of a patient's blood vessel.
39. The intravascular blood pump according to claim 38, wherein the anchor comprises spikes that are expandable and collapsible.
40. The intravascular blood pump according to any of claims 1 to 39, comprising an anchoring structure at a distal end region of the intravascular blood pump.
41. The intravascular blood pump according to paragraph 40, wherein the anchoring structure comprises a hook or loop at an axial end of the distal end region.
42. The intravascular blood pump according to paragraph 41, wherein the loop is made from a soft, elastic material and forms an atraumatic distal extension at the axial end of the intravascular blood pump.
43. The intravascular blood pump according to paragraph 41, wherein the loop is provided at an atraumatic distal extension of the intravascular blood pump.
44. The intravascular blood pump according to any one of paragraphs 40 to 43, wherein the anchoring structure comprises a neck-and-head structure at an axial end of the distal end region.
45. The intravascular blood pump according to paragraph 44, wherein a connecting surface between a head and a neck of the neck-and-head structure is inclined by 90° relative to a longitudinal axis of the intravascular blood pump, or the connecting surface is inclined by more than 90° relative to the longitudinal axis of the intravascular blood pump so as to form an undercut.
46. The intravascular blood pump according to paragraph 44 or 45, wherein the neck-and-head structure is formed in a recess of the distal end region.
47. The intravascular blood pump according to paragraph 44 or 45, wherein the neck-and-head structure is formed at an end of an atraumatic distal extension.
48. An introducer set, comprising an intravascular blood pump according to any one of paragraphs 40 to 47 and a connecting catheter which comprises a connector adapted to connect to the anchoring structure of the intravascular blood pump.
49. The introducer set according to paragraph 48, wherein the connector comprises a hook adapted to hook into the anchoring structure of the intravascular blood pump.
50. The introducer set according to paragraph 48, wherein the connector comprises a gripper having a first grip and a second grip adapted to close or grip around the hook or loop of the intravascular blood pump.
51. The introducer set according to paragraph 50, wherein the gripper has a gripping surface, wherein the gripping surface is inclined by 90° relative to a longitudinal axis of the connecting catheter.
52. The introducer set according to paragraph 50, wherein the gripper has a gripping surface, wherein the gripping surface is inclined by more than 90° relative to the longitudinal axis of the connecting catheter so as to form an undercut.
53. A method of placing an intravascular blood pump in a patient, the blood pump comprising a pump section having a blood flow inlet and a blood flow outlet and an impeller rotatable about an axis of rotation and sized and shaped for conveying blood from the blood flow inlet to the blood flow outlet, the blood pump further comprising a supply line, preferably a supply catheter, connected to the pump section and adapted to supply energy, preferably electric energy, for driving the impeller, the method comprising the steps of:
   - placing a first guide wire through the patient's vascular system, including through the patient's heart, such that it extends out of the patient's body with a first end through a first percutaneous access on an arterial side of the vascular system, preferably through the axillary artery or subclavian artery, and with a second end through a second percutaneous access on a venous side of the vascular system, preferably through the subclavian vein or axillary vein, and
   - using the first guide wire in the further procedure of placing the intravascular blood pump into the patient's body through the second percutaneous access.
54. The method of paragraph 53, comprising one or more of the following steps:
   - inserting a transseptal sheath from the venous side of the patient's vascular system through a passage created in the septum of the patient's heart into the left side of the heart, and advancing the first end of the first guide wire through the transseptal sheath into the left part of the heart and pulling back the transseptal sheath,
   - wherein preferably the transseptal sheath is inserted through a third percutaneous access into a vein below the vena cava inferior, preferably a femoral vein, and further comprising the steps of: inserting a snare catheter through the second percutaneous access into a vein above the vena cava superior, advancing the snare catheter into the vena cava superior towards the first guide wire, catching the first guide wire with the snare catheter, and moving the second end of the first guide wire into the third percutaneous access and out of the second percutaneous access with the aid of the snare catheter,
   - expanding the passage through the septum, preferably using a balloon catheter,
   - advancing an introducer sheath through the septum, preferably with the aid of the balloon catheter, such that a front part of the introducer sheath extends into the left part of the heart, preferably into the left ventricle,
   - withdrawing the balloon catheter, where applicable,
   - advancing a maneuverable catheter, preferably a Swan-Ganz catheter, through the introducer sheath into the left part of the heart, preferably into the left ventricle,
   - preferably, advancing a second guide wire through the first percutaneous access into the left part of the heart, preferably into the left ventricle, and
   - guiding the first end of the first guide wire towards and out of the first percutaneous access with the aid of the maneuverable catheter, preferably by inflating a balloon of the Swan-Ganz catheter and following the patient's blood flow, preferably along a direction which is indicated by the second guide wire.
55. The method of paragraph 54, comprising one or more of the following steps:
   - attaching, preferably in a form-fitting manner, a front end of a coupling catheter to the first end of the first guide wire and guiding the coupling catheter through the patient's vascular system, including through the patient's heart, using the first guide wire until the front end of the coupling catheter extends through the second percutaneous access out of the patient's body, preferably while simultaneously withdrawing the maneuverable catheter from the patient's body,
   - coupling a distal end of the intravascular blood pump to the coupling catheter, preferably in a form-fitting manner, and advancing the intravascular blood pump through the patient's vascular system, including through the septum, so as to place the pump section into the left part of the heart, preferably across the aortic valve,
   - preferably, anchoring the intravascular blood pump in a wall of a blood vessel, preferably in an aortic wall, preferably by moving a plurality of spikes from a circumference of the intravascular blood pump from a radially collapsed configuration to a radially expanded configuration to engage the wall of the blood vessel,
   - decoupling the coupling catheter from the distal end of the intravascular blood pump and withdrawing the coupling catheter from the patient's body, and
   - withdrawing the introducer sheath from the patient's body.
56. The method of paragraph 54, comprising one or more of the following steps:
   - withdrawing the maneuverable catheter from the patient's body while keeping the first end of the first guide wire extending through the second percutaneous access out of the patient's body,
   - guiding the introducer sheath along the first guide wire through the patient's vascular system, including through the patient's heart, until the front end of the introducer sheath extends through the second percutaneous access out of the patient's body,
   - feeding the second end of the first guide wire through a loop provided at a distal end of the intravascular blood pump and advancing the second end of the first guide wire through the introducer sheath until the second end of the first guide wire extends out of the front end of the introducer sheath,
   - gripping the first end second ends of the first guide wire which extend through the second percutaneous access out of the introducer sheath and advancing the intravascular blood pump along the introducer sheath through the patient's vascular system, including through the septum, so as to place the pump section into the left part of the heart, preferably across the aortic valve,
   - releasing or unfeeding the first guide wire from the loop provided at the distal end of the intravascular blood pump and withdrawing the first guide wire from the introducer sheath,
   - withdrawing the introducer sheath from the patient's body, and
   - preferably, anchoring the intravascular blood pump in a wall of a blood vessel, preferably in an aortic wall, preferably by moving a plurality of spikes from a circumference of the intravascular blood pump from a radially collapsed configuration to a radially expanded configuration to engage the wall of the blood vessel.
57. A method of placing an intravascular blood pump in a patient, the blood pump comprising a pumping device, the pumping device comprising a pump section having a blood flow inlet and a blood flow outlet and an impeller rotatable about an axis of rotation and sized and shaped for conveying blood from the blood flow inlet to the blood flow outlet, and a drive section connected to the pump section and adapted to drive the impeller, the blood pump further comprising a supply line, preferably a supply catheter, connected to the pumping device and adapted to supply the drive section at least with energy, preferably electric energy, for driving the impeller, the method comprising the steps of:
   - accessing the patient's thoracic cavity;
   - creating a puncture through an apical wall of the heart of the patient to access the left ventricle of the heart;
   - advancing the pumping device through the puncture in the apical wall such that the blood flow inlet of the pump section is disposed in the left ventricle of the heart, the blood flow outlet of the pump section is disposed in the patient's aorta, the supply line extends through the puncture in the apical wall out of the patient's body, and the drive section is disposed in the aorta.
58. The method of paragraph 57, comprising the step of anchoring the intravascular blood pump in a wall of a blood vessel, preferably in an aortic wall, preferably by moving a plurality of spikes from a circumference of the intravascular blood pump from a radially collapsed configuration to a radially expanded configuration to engage the wall of the blood vessel.
59. A kit for placing an intravascular blood pump in a patient comprising the following tools:
   - a transseptal sheath (17), which may include a puncture tip end or separate puncture needle and a dilator,
   - a first guide wire (20A),
   - a second guide wire (20B),
   - an introducer sheath (19),
   - a snare catheter (18),
   - a balloon catheter (22),
   - a maneuverable catheter (23),
   - either a connecting catheter (25) or a tube (24).

## Claims

1. An intravascular blood pump for percutaneous insertion into a patient's vasculature, the intravascular blood pump comprising a pumping device (30) and a supply line (36),
- wherein the pumping device (30) comprises a pump section (32) having a blood flow inlet (33), a blood flow outlet (34) and an impeller rotatable about an axis of rotation for conveying blood from the blood flow inlet to the blood flow outlet, and a drive section (31) connected to the pump section (32) and adapted to drive the impeller, and
- wherein the supply line (36) is adapted to supply energy for driving the impeller, and
- wherein an anchoring structure (60; 70) is provided at a distal end region of the intravascular blood pump.

2. The intravascular blood pump according to claim 1, wherein the anchoring structure comprises at least one anchor adapted to anchor the intravascular blood pump in a wall of a patient's blood vessel.

3. The intravascular blood pump according to claim 2, wherein the anchor (41) comprises spikes that are expandable and collapsible.

4. The intravascular blood pump according to any one of claims 1 to 3, wherein the anchoring structure comprises a hook or loop (60) at an axial end (30A) of the distal end region.

5. The intravascular blood pump according to claim 4, wherein the loop (60) is made from a soft, elastic material and forms an atraumatic distal extension at the axial end of the intravascular blood pump.

6. The intravascular blood pump according to claim 4, wherein the loop (60) is provided at an atraumatic distal extension (40) of the intravascular blood pump.

7. The intravascular blood pump according to any one of claims 1 to 3, wherein the anchoring structure comprises a neck-and-head structure at an axial end (30A) of the distal end region.

8. The intravascular blood pump according to claim 7, wherein a connecting surface (72) between a head (70) and a neck (71) of the neck-and-head structure is inclined by 90° relative to a longitudinal axis of the intravascular blood pump, or the connecting surface (72) is inclined by more than 90° relative to the longitudinal axis of the intravascular blood pump so as to form an undercut.

9. The intravascular blood pump according to claim 7 or 8, wherein the neck-and-head structure is formed in a recess of the end region.

10. The intravascular blood pump according to claim 7 or 8, wherein the neck-and-head structure is formed at an end of an atraumatic distal extension (40).

11. An introducer set, comprising an intravascular blood pump according to any one of claims 1 or 4 to 10 and a connecting catheter (25) which comprises a connector adapted to connect to the anchoring structure (60; 70) of the intravascular blood pump.

12. The introducer set according to claim 11, wherein the connector comprises a hook adapted to hook into the anchoring structure of the intravascular blood pump.

13. The introducer set according to claim 11, the connector comprises a gripper having a first grip (26A) and a second grip (26B) adapted to close or grip around the anchoring structure of the intravascular blood pump.

14. The introducer set according to claim 13, wherein the gripper has a gripping surface (26C), wherein the gripping surface is inclined by 90° relative to a longitudinal axis of the connecting catheter.

15. The introducer set according to claim 13, wherein the gripper has a gripping surface (26C), wherein the gripping surface is inclined by more than 90° relative to the longitudinal axis of the connecting catheter so as to form an undercut.

16. A kit for placing an intravascular blood pump in a patient comprising the following tools:
- a transseptal sheath (17), which may include a puncture tip end or separate puncture needle and a dilator,
- a first guide wire (20A),
- a second guide wire (20B),
- an introducer sheath (19),
- a snare catheter (18),
- a balloon catheter (22),
- a maneuverable catheter (23),
- either a connecting catheter (25) or a tube (24).
